# EUROPEAN PATENT APPLICATION

(11) **EP 1 323 420 A1**
(43) Date of publication of application: **02.07.2003**
(21) Application number: 01972601.7
(22) Date of filing: 28.09.2001
(51) Int. Cl.: A61K 31/4245, A61K 31/4355, A61K 31/4365, A61K 31/437, A61K 31/4439, A61K 31/4725, A61K 31/513, A61K 31/5377, A61K 31/55, A61K 38/05, A61P 1/00, A61P 1/04, A61P 43/00, C07D 413/12

(54) **REMEDIES FOR INFLAMMATORY BOWEL DISEASES**

(30) Priority: 29.09.2000 JP 2000297879
(71) Applicant: ONO PHARMACEUTICAL CO., LTD., Osaka-shi, Osaka 541-8526 (JP)
(72) Inventor: HIROTA, Yasushi, Sakai-gun, Fukui 913-0032 (JP)
(74) Representative: Henkel, Feiler, Hänzel
(86) International application number: JP0108570
(87) International publication number: WO02026230

(57) **Abstract**

Prophylactic and/or therapeutic drugs against ulcerative colitis, which include five-member heterocyclic compounds represented by the formula (I) or these nontoxic salts as active principle, 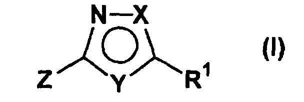 (wherein Z represents the group containing α-aminocarbonyl group, R¹ represents (1) substituted alkyl, alkenyl or alkynyl groups, (2)OH, (3)amino, (4)alkylamino, (5)dialkylamino, etc., X and Y represent oxygen atom, sulfur atom or substituted nitrogen atom.)

## Description

### TECHNICAL FIELD

The present invention relates to medicines of ulcerative colitis.

More particularly, the present invention relates to, 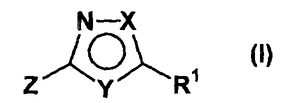 medicines of ulcerative colitis, which include five-member heterocyclic compounds represented by the formula (I) (all signs in the formula represent the same meanings as postscripts.) and have inhibitory activity against elastase or these nontoxic salts as active principle.

### BACKGROUND OF THE INVENTION

Human neutrophil elastase (HNE) is a kind of serine protease, and protein proteinase that is secreted from neutrophils by various inflammatory stimulus and takes a part in connective tissue biolysis. HNE activity is regulated by alpha1-proteinase inhibitor that is inhibitory factor in vivo, and the symptom of tissue destruction appears when unbalance between the enzyme and the inhibitor arise. For example, it is well known that it is involved in pulmonary emphysema, atherosclerosis and arthritis, etc.

Ulcerative colitis is a chronic disease of which intestines brought about inflammation, for instance, ulcerative colitis, Crohn's disease, the ischemic colitis, and the intestinal Behcet's syndrome, etc. are enumerated.

Ulcerative colitis starts by the degeneration of net fibre under the mucosal epithelium, and the blockage of the capillary and the progressive invasion of eosinophils or lymphocytes, and then forms the festering and the ulcer. Though the cause are thought to be the bacterial infection or the cytotoxic action of the mucus dialytic ferment or the reproductive function of the mucous membrane or allergic reaction to diet, etc., anything are thought to be not primary cause, but to act making to severity and chronicity of this symptom, the cause has not been elucidated yet. Usually, this symptom repeatedly makes to aggravation and abatement, and then makes to chronicity. The change to a morbid state starts at the rectum and under the sigmoid colon, and then limits in these part or extends to proximal and finally violates all colons.

The main symptom is diarrhea, and bloody feces. Moreover, the constitutional symptom of pyretic, anemia, anorexia, loss of weight, hyperleukocytosis, and hypoalbuminemia, etc. comes to be shown. Though salazosulfapyridine and the drenocortical steroid are chiefly used now as the drug therapy, it cannot be said to be enough because of problems of the side effects.

Crohn's disease first presents the crypt flame and crypt cystoma, and then progresses to the ulcer. Moreover, it might form granuloma, and progresses to making to fiber consequentially. However, these fundamental causes have not been understood yet. It is indicated that the genetic factor causes undesirable adjustment of the immune reaction of intestines against the environment, diet, and the source of infection now.

This disease is called a regional enteritis clearly distinguished from normal intestines. The lesioned part appears only in the ileum or the colon, or both and often appears especially right in the colon. Moreover, it might appear in the anus surroundings, or, in addition, all small intestines be violated. The main symptom is the chronic diarrhea with stomachache, heat, anorexia, loss of weight or tumor and feeling of fullness in right lower quadrant of abdomen. Though diphenoxylate, cholilytic drug, antibiotic, salazosulfapyridine, and drenocortical steroid are chiefly used now as the drug therapy, it cannot be said to be enough because of problems of the side effects.

The ischemic colitis is caused by the ischemia of intestines caused in the failure of vascular which sends blood to intestines. This disease often causes along with arteriosclerotic, and mostly causes transitional regional inflammations. It usually develops in a left colon, and presents congestion, the hemorrhage, the multiple sore, and the longitudinal ulcer, etc. The intestinal might be necrosis when getting a serious illness.

The intestinal Behcet's syndrome is an inflammatory and recidivated chronic disease of which cause is uncertain and first presents the ulcer. It presents various symptoms, up to the syndrome like Crohn's disease from nonspecific abdominal discomfort.

There are reports such as the following about elastase and ulcerative colitis. It has been reported that the elastase activity in irrigation water of the intestinal diverticulum of ulcerative colitis patient rises [Eur. J. Gastroenterol Hepatol, 12(5), 553-557 (2000)], and elastase in the plasma rises [Hepatogastroenterology, 46(28), 2315-2320 (1999)]. Moreover, it has been reported that elastase rises in ulcerative colitis patient's feces [Medicina (B Aires), 58(3), 262-264 (1998)].

However, there is no report that colitis is caused by activating elastase or deteriorates, etc.

### DISCLOSURE OF THE INVENTION

As a result that the present inventors examined assiduously under the above conditions, 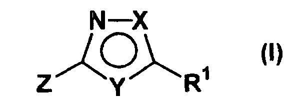 it was first found that serine protease inhibitors, especially five-member heterocyclic compounds represented by the formula (I) (all signs in the formula show the same meanings as postscripts.), with inhibitory activity against elastase, or these nontoxic salts are effective in ulcerative colitis, and the present invention was completed.

That is, the present invention relates to, 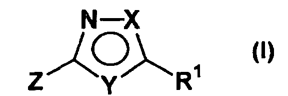 (wherein Z represents the group to contain α-aminocarbonyl group that the carbonyl carbon atom combines with carbon atom of heterocyclic group through covalent bond,

R¹ represents (1) alkyl, alkenyl or alkynyl groups, with the proviso that these groups may be substituted with one or more groups chosen from (a)halogen atom, (b)hydroxy, (c)cyano, (d)nitro, (e)haloalkyl, (f)alkylamino, (g)dialkylamino (h)alkoxy, (l)haloalkoxy, (j)carboxy, (k)carboalkoxy, (l)alkylcaroxamide, (m)arylcaroxamide or (n)-O-(C5-C6) aryl groups,
(2)hydroxy,
(3)amino,
(4)alkylamino,
(5)dialkylamino, or
(6)cycloalkyl, alkylcycloalkyl, alkenylcycloalkyl, cycloalkenyl, alkylcycloalkenyl, alkenylcycloalkenyl, (C5-C12)aryl, (C5-C12)arylalkyl, (C5-C12)arylalkenyl, condensable (C5-C12)aryl-cycloalkyl or alkyl condensable (C5-C12)aryl-cycloalkyl, with the proviso that these groups may be substituted with groups chosen from (a)halogen atom, (b)hydroxy, (c)cyano, (d)nitro, (e)haloalkyl, (f)amino, (g)aminoalkyl, (h)dialkylamino, (i)alkyl, (j)alkenyl, (k)alkylenedioxy, (l)alkynyl, (m)alkoxy, (n)haloalkoxy, (o)carboxy, (p)carboalkoxy, (q)carboxamide, (r)(C5-C6)aryl, (s)-O-(C5-C6)aryl, (t)arylcarboxamide, (u)alkylthio or (v)haloaklylthio that may include 1-4 of hetero atoms chosen from nitrogen, oxygen or sulfur atom, respectively,
X and Y each independently represent oxygen, sulfur, nitrogen or atom,
which nitrogen atom may be substituted with groups chosen from
(1)alkyl or alkenyl that may be substituted with 1-3 of halogen atom,
(2)alkynyl,
(3)(C5-C6)aryl, arylalkyl, arylalkenyl that may include 1-3 of hetero atoms chosen from nitrogen, oxygen or sulfur atom or may be substituted with groups chosen from (a)halogen atom, (b)cyano, (c)nitro, (b)hydroxy, (e)haloalkyl, (f)amino, (g)aminoalkyl, (h)dialkylamino, (i)alkyl, (j)alkenyl, (k)alkynyl, (l)alkoxy, (m)haloalkoxy, (n)carboxy, (o)carboalkoxy, (p)carboxamide, (q)arylcarboxamide, (r)alkylthio or (s)haloaklylthio);
prophylactic drugs and/or medicines that include five-member heterocyclic compounds represented by formula (I) or these nontoxic salts as active principle for ulcerative colitis.

These compounds represented by formula (I) are mentioned in WO96/16080 and WO98/24806 and are mentioned that they could be used for therapy and/or prophylaxis of adult respiratory distress syndrome, septicemic shock, multiple organopathy, myocardial ischemia, reperfusion injury, emphysema, arthritis, periodontal disease, nephritis, dermatitis, psoriasis, cystic fibrosis, chronic bronchitis, atherosclerosis, Alzheimer's disease, organ transplant, corneal ulcer and invasion of malignant tumor. However, there is no mention related to asthma and restrainers of the airway mucus secretion in the above specifications, of course, the validity isn't confirmed. If they don't try with the pathological model of the applicable disease, it doesn't know whether compounds are effective in the sickness.

Z in compounds shown in the formula (I) used for the present invention represents the group containing carbonyl group.

In compounds represented by the formula (I), compounds that Z is represented by some of the following groups chosen from the formula (I-1) to (I-4), or these non-toxic salts are desirable.

More particularly, in compounds represented by the formula (I), the compound represented by the formula (I) of which Z is represented
by the formula (I-1), 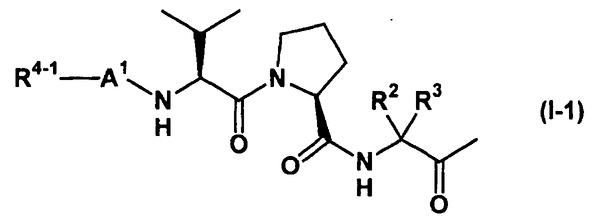 (wherein R² and R³ each independently represent alkyl, alkenyl, -RCOR', -RCOOR', -RNR'R"R°, or -RC(O)NR'R" [wherein R represents alkyl or alkenyl, R', R" and R° each independently represent hydrogen atom, alkyl, alkenyl, cycloalkyl or (C5-C6)aryl.] with the proviso that the above groups optionally be substituted with hydrogen, 1-3 of halogen atom, hydroxy, sulfur atom, alkylthio, amino, alkylamino, dialkylamino, alkylguanidinyl, dialkylguanidinyl, guanidinyl or amidylguanidine; or cycloalkyl, alkylcycloalkyl, alkenylcycloalkyl, alkyl-oxyaryl, alkyl-thioaryl, alkyl-aminoaryl, (C5-C12)aryl, (C5-C12)arylalkyl or (C5-C12)arylalkenyl with the proviso that the above groups optionally include 1-4 of hetero atoms chosen from nitrogen, oxygen or sulfur atom and are substituted with halogen atoms, cyano, keto, nitro, hydroxy, haloalkyl, amino, aminoalkyl, dialkylamino, amidine, alkylamidine, dialkylamidine, alkyl, alkenyl, alkylenedioxy, alkynyl, alkoxy, haloalkoxy, carboxy, carboalkoxy, alkylcarboxamide, (C5-C6)aryl, -O-(C5-C6)aryl, arylcarboxamide, alkylthio or haloalkylthio,
A¹ represents direct coupling, -C(O)-, -NH-C(O)-, -S(O)₂-, -NH-S(O)₂-, -C(O)-, -C- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom,
R⁴⁻¹ represents cycloalkyl, alkylcycloalkyl, (C5-C12)aryl, (C5-C12)arylalkyl, condensed (C5-12)aryl-cycloalkyl or condensed alkyl(C5-C12)aryl-cycloalkyl with the proviso that these groups optionally include hydrogen atom, alkyl, alkenyl or alkynyl, one or more hetero atoms chosen from nitrogen, oxygen or sulfur atom, and optionally be substituted with alkyl, alkenyl, alkynyl, halogen atom, cyano, nitro, hydroxy, haloalkyl, alkoxy, amino, aminoalkyl, dialkylamino, carboxy, haloalkoxy, carboalkoxy, alkylcarboxamide, aryl, arylalkyl, arylcarboxamide, alkylthio or haloalkylthio);
the formula (I-2). 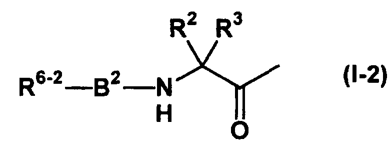 (wherein R² and R³ represent the same meanings as aforesaid;
B² represents -S(O)₂-, -C(O)-, -OC(O)- or -CH₂C(O)-;
R⁶⁻² represents the following groups; 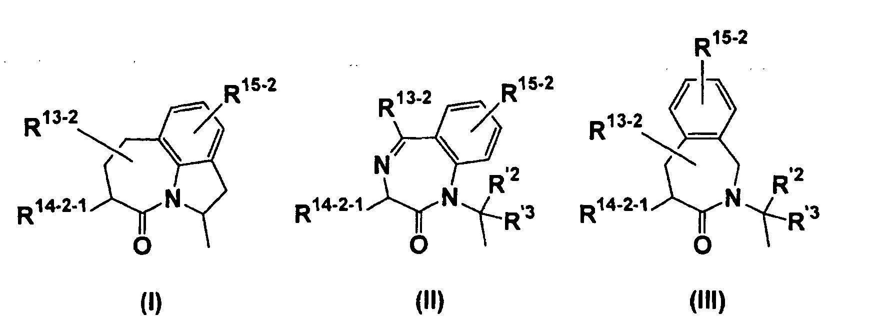 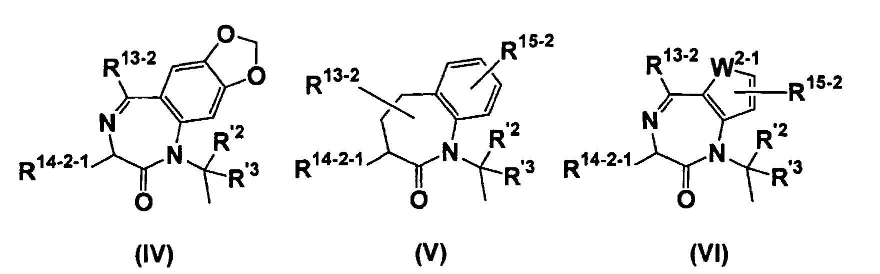 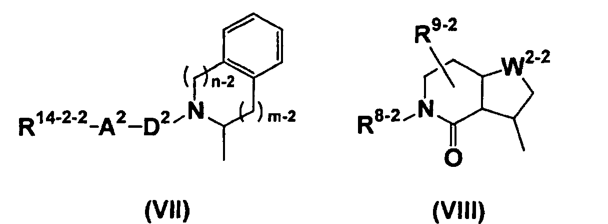 (wherein R'² and R'³ represent the same meanings as R² and R³;
R¹³⁻² represents aryl, arylalkyl, cycloalkyl, alkylcycloalkyl, condensed aryl-cycloalkyl, alkyl condensed aryl-cycloalkyl with the proviso that these groups optionally include hydrogen atom, alkyl, halogen atom, alkoxy, carboalkoxy, carboxy, alkylthio, amino, alkylamino, dialkylamino or one or more hetero atoms chosen from nitrogen, oxygen or sulfur atom, and optionally be substituted with halogen atom or alkyl;
R¹⁴⁻²⁻¹ represents aryl, arylalkyl, cycloalkyl, alkylcycloalkyl, condensed aryl-cycloalkyl, alkyl condensed aryl-cycloalkyl or aryloxycarboxamide with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, arylalkylcarboxamide, alkylthio or haloalkylthio;
R¹⁵⁻² represents aryl, arylalkyl, cycloalkyl, alkylcycloalkyl, condensed aryl-cycloalkyl or alkyl condensed aryl-cycloalkyl with the proviso that these groups optionally include halogen atom, alkyl, halogen atom, alkoxy, carboalkoxy, carboxy, alkylthio, amino, alkylamino, dialkylamino or one or more hetero atoms chosen from nitrogen, oxygen or sulfur atom;
W²⁻¹ represents oxygen or sulfur atom; carbon or nitrogen atom that optionally be substituted with hydrogen atom, alkyl or aryl;
[m-2] represents 0 or 1;
[n-2] represents 0 or 1;
D² represents direct coupling or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A² represents direct coupling, -C(O)-, -NH-C(O)-, -S(O)₂-, -NH-S(O)₂-, -C(O)- or -C-;
R¹⁴⁻²⁻² represents aryl, arylalkyl, cycloalkyl, alkylcycloalkyl, condensed aryl-cycloalkyl or alkyl condensed aryl-cycloalkyl with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio;
W²⁻² represents sulfur or oxygen atom;
R⁸⁻² represents alkylamino dialkylamino or amino;
R⁹⁻² represents hydrogen atom, alkyl or halogen atom));
the formula (1-3), 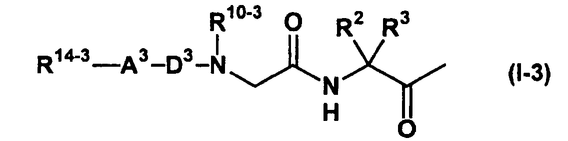 (wherein R² and R³ represent the same meanings as aforesaid,
R¹⁰⁻³ represents (C5-C6)aryl, (C5-C6)arylalkyl, (C5-C6)arylalkenyl, cycloalkyl, condensed aryl-cycloalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, alkylcarboxamide, alkylthio or haloalkylthio;
D³ represents direct coupling, -C(O)- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A³ represents direct coupling, -C(O)-, -NH-C(O)-, -S(O)₂-, -NH-S(O)₂-, -C(O)-, -S(O)2-NH-, OC(0)NH-, -OC(O)- or -C-;
R¹⁴⁻² and R¹⁴⁻²⁻¹ represent the same meanings as aforesaid),
the formula(I-4); 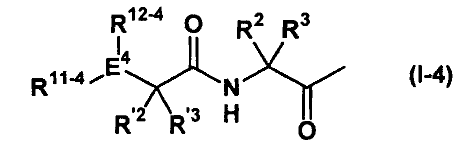 (wherein R², R³ R'² and R'³ represent the same meanings as aforesaid,
R¹¹⁻⁴, R¹²⁻⁴ and E⁴ are constituted of monocyclic or dicyclic ring with the proviso that these rings include 5-10 atoms chosen from carbon, nitrogen, sulfur or oxygen atom and include one or more keto groups and optionally be substituted with halogen atom, cyano, nitro, haloalkyl, amino, aminoalkyl, dialkylamino, alkyl, alkenyl, alkynyl, alkoxy, haloalkoxy, carboxy, carboalkoxy, alkylcarboxamide, alkylthio or haloalkylthio; and represent cycloalkyl, alkylcycloalkyl, alkenylcycloalkyl, (C5-C12)aryl, (C5-C12)arylalkyl, ((C5-C12)arylalkyl)OC(O)NH- or (C5-C12)arylalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, -C(O)O(alkyl), -C(O)(alkyl), alkylcarboxamide, alkylthio or haloalkylthio) or these nontoxic salts are desirable.

Further, in the above formula(I-4), R¹¹⁻⁴, R¹²⁻⁴ and E⁴ are constituted of a ring, respectively. Concretely, for R¹¹⁻⁴, R¹²⁻⁴ and E⁴, groups chosen from the following formula(I-4-1) to formula(I-4-13) are given.

If Z in compounds represented by the formula(I) of the present invention is a group represented by the formula(I-4), these compounds that R¹¹⁻⁴, R¹²⁻⁴ and E⁴ are represented by groups chosen from the following formula(I-4-1) to formula(I-4-13) or these nontoxic salts are desirable.

More particularly, if Z in compounds represented by the formula(I) of the present invention is a group represented by the formula(I-4), compounds represented by the formula (I) that R¹¹⁻⁴, R¹²⁻⁴ and E⁴ are represented
by the formula (I-4-1), 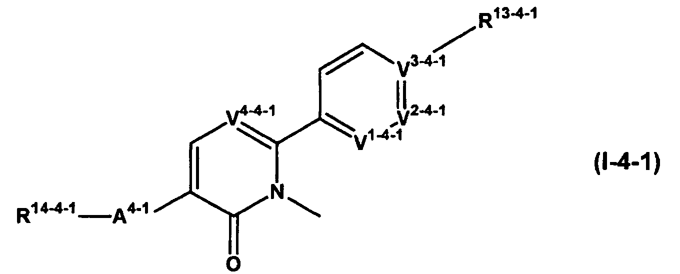 (wherein A⁴⁻¹ represents the same meanings as A³,
V¹⁻⁴⁻¹, V²⁻⁴⁻¹, V³⁻⁴⁻¹ and V⁴⁻⁴⁻¹ each independently represent carbon or nitrogen atom, in case V³⁻⁴⁻¹ represents carbon atom, R¹³⁻⁴⁻¹ represents hydrogen atom, alkyl, halogen atom, alkoxy, carboalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom, and optionally be substituted with halogen atom or alkyl;
R¹⁴⁻⁴⁻¹ represents hydrogen atom, alkyl, alkenyl, amino, alkylamino or dialkylamino; aryl, arylalkyl, cycloalkyl, alkylcycloalkyl, condensed aryl-cycloalkyl or alkyl condensed aryl-cycloalkyl, arylalkylcarbonyl or arylalkylcarboxamide with the proviso that these groups optionally include one or more hetero atoms-chosen-from-nitrogen, sulfur or oxygen- atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio), the formula (I-4-2), 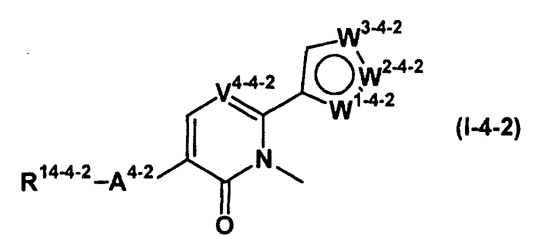 (wherein R¹⁴⁻⁴⁻² represents the same meanings as R¹⁴⁻⁴⁻¹,
A⁴⁻² represents the same meanings as A⁴⁻¹,
V⁴⁻⁴⁻² represents the same meanings as A⁴⁻⁴⁻¹,
W¹⁻⁴⁻², W²⁻⁴⁻² and W³⁻⁴⁻² each independently represent nitrogen, carbon or oxygen atom that optionally be substituted with alkyl),
the formula (I-4-3). 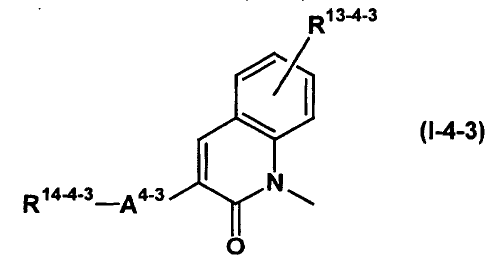 (wherein R¹⁴⁻⁴⁻³ represents the same meanings as R¹⁴⁻⁴⁻¹,
A⁴⁻³ represents the same meanings as A⁴⁻¹,
R¹³⁻⁴⁻³ represents the same meanings as R¹³⁻⁴⁻¹),
the formula (I-4-4), 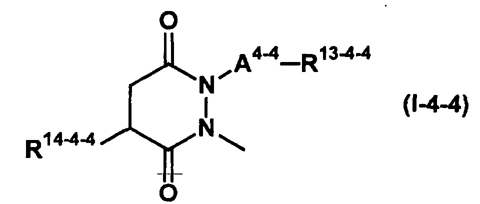 (wherein A⁴⁻⁴ represents direct coupling, -C- or -C(O)-,
R¹³⁻⁴⁻⁴ represents the same meanings as R¹³⁻⁴⁻¹,
R¹⁴⁻⁴⁻⁴ represents the same meanings as R¹⁴⁻⁴⁻¹),
the formula (I-4-5), 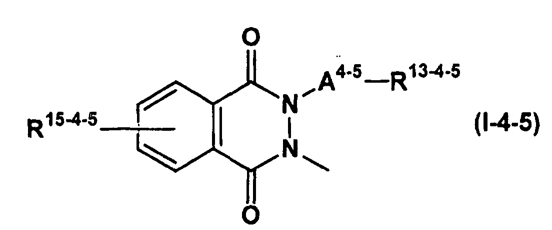 (wherein A⁴⁻⁵ represents the same meanings as A⁴⁻⁴,
R¹³⁻⁴⁻⁵ represents the same meanings as R¹³⁻⁴⁻¹,
R¹⁵⁻⁴⁻⁵ represents the same meanings as R¹⁵⁻²),
the formula (I-4-6), 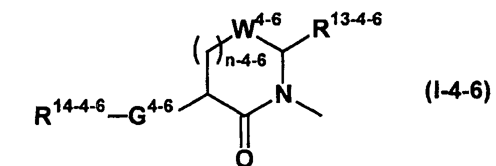 (wherein W⁴⁻⁵ represents sulfur atom, SO, SO₂, or carbon atom,
[n-4-6] represents zero, one or two,
R13-4-6 represents the same meanings as R¹³⁻⁴⁻¹,
R¹⁴⁻⁴⁻⁶ represents the same meanings as R¹⁴⁻⁴⁻¹,
G⁴⁻⁶ represents -NHC(O)-, -OC(O)NH-, -C(O)-, -NHS(O)₂- or direct coupling), 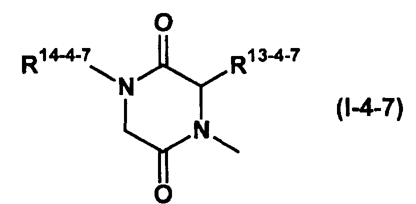 (wherein R¹³⁻⁴⁻⁷ represents the same meanings as R¹³⁻⁴⁻¹, or represents CH=R¹⁵⁻⁴⁻⁷ or R¹⁵⁻⁴⁻⁷,
R¹⁵⁻⁴⁻⁷ represents pyridinyl, phenyl or benzyl that optionally be substituted with halogen atom, dialkylamino or -C(O)OCH₂,
R¹⁴⁻⁴⁻⁷ represents hydrogen atom, alkyl, alkenyl, CH₂C(O)-; aryl, arylalkyl, cycloalkyl, alkylcycloalkyl, condensed aryl-cycloalkyl or alkyl condensed aryl-cycloalkyl, aryloxycarbonyl or arylalkyloxycarbonyl with the proviso that these groups optionally include one or more hetero atoms chosen from nitrogen, sulfur or oxygen atom, and optionally be substituted with alkyl, halogen atom, alkoxy, amino, alkylamino, dialkylamino, carboxy, alkenyl, haloalkoxy, carboalkoxy, alkylcarboxamide, aryl, arylalkyl, arylcarboxamide, alkylthio or haloalkylthio),
the formula (I-4-8), 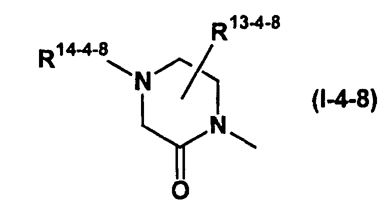 (wherein R¹³⁻⁴⁻⁸ represents the same meanings as R¹³⁻⁴⁻⁷,
R¹⁴⁻⁴⁻⁸ represents the same meanings as R¹⁴⁻⁴⁻⁷),
the formula (I-4-9), 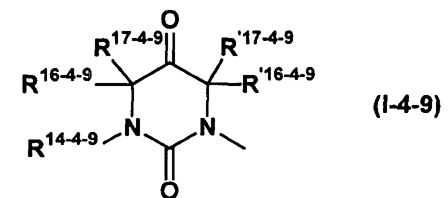 (wherein R¹⁴⁻⁴⁻⁹ represents the same meanings as R¹⁴⁻⁴⁻⁷,
R¹⁶⁻⁴⁻⁹, R¹⁷⁻⁴⁻⁹, R'¹⁶⁻⁴⁻⁹ and R'¹⁷⁻⁴⁻⁹ each independently represent hydrogen atom, alkyl, alkenyl, alkylthio, alkylthioalkyl or guanidine; cycloalkyl, cycloalkeny, alkylcycloalkyl, aryl, arylalkyl or arylalkenyl that optionally be substituted with carboalkoxy, hydroxy, haloalkyl, alkylthio, alkylguanidine, dialkyl guanidine or amidine),
the formula (I-4-10). 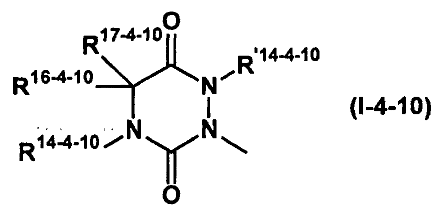 (wherein R¹⁴⁻⁴⁻¹⁰ and R'¹⁴⁻⁴⁻¹⁰ each independently represent the same meanings as R¹⁴⁻⁴⁻⁷,
R¹⁵⁻⁴⁻¹⁰ represents the same meanings as R¹⁵⁻⁴⁻⁹,
R¹⁷⁻⁴⁻¹⁰ represents the same meanings as R¹⁷⁻⁴⁻⁹),
the formula (I-4-11). 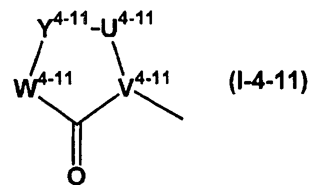 [wherein U⁴⁻¹¹, V⁴⁻¹¹, W⁴⁻¹¹ and Y⁴⁻¹¹ each independently represent nitrogen, carbon atom, C(O), N(R¹³⁻⁴⁻¹¹) (wherein R¹²⁻⁴⁻¹¹ represents hydrogen atom, alkyl, halogen atom, alkoxy, carbalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom and optionally be substituted with halogen atom or alkyl.), N(R¹⁴⁻⁴⁻¹¹) (wherein R¹⁴⁻⁴⁻¹¹ represents hydrogen atom, alkyl, alkenyl; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio.) or C(R¹⁵⁻⁴⁻¹¹)(R¹⁷⁻⁴⁻¹¹) (wherein R¹⁵⁻⁴⁻¹¹ and R¹⁷⁻⁴⁻¹¹ each independently are constituted of hydrogen atom, alkyl, alkylthio, alkylthioalkyl, carboxylate represented by the for formula -(CH₂)ₘ₋₄₋₁₁C(O)OR⁰⁻⁴⁻¹¹ or N-substituted alkylamide presented by -(CH₂)ₘ₋₄₋₁₁C(O)NR⁰⁻⁴⁻¹¹R'⁰⁻⁴⁻¹¹ (wherein [m-4-11] represents an integer of 1 to 6, and R0-4-11 and R'0-4-11 each independently represent aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or non-peroxideone oxygen atom and optionally be substituted with amino, alkylamino, dialkylamino, guanidine, carboalkoxy, keto, hydroxy, alkyl, haloalkyl, alkylthio, alkylguanidine, dialkylguanidine or amidine.) or rings that include 4-8 atoms chosen from carbon, nitrogen, oxygen atom or sulfur atom.)],
the formula (I-4-12), 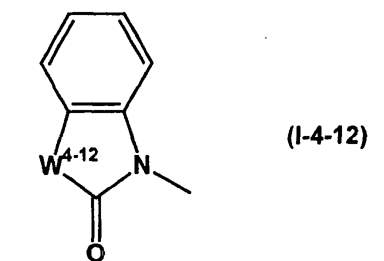 (wherein W⁴⁻¹² represents the same meanings as W⁴⁻¹¹),
the formula (I-4-13), 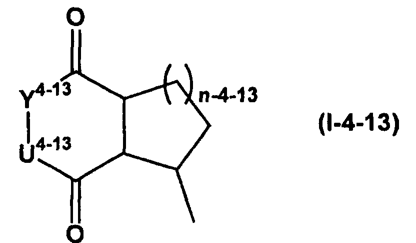 [wherein U⁴⁻¹² and V⁴⁻¹² each independently represent nitrogen, carbon atom, N(R¹³⁻⁴⁻¹³) or C(R¹⁶⁻⁴⁻¹³)(R¹⁷⁻⁴⁻¹³) (wherein R¹³⁻⁴⁻¹³ represents hydrogen atom, alkyl, alkoxy, carbalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from oxygen, nitrogen or sulfur atom, and R¹⁶⁻⁴⁻¹³ represents the same meanings as R¹⁶⁻⁴⁻¹¹,
R¹⁷⁻⁴⁻¹³ represents the same meanings as R¹⁶⁻⁴⁻¹¹),
[n-4-13] represents 1 or 2.] or these nontoxic salts are desirable.

In compounds represented by the formula(I) of the present invention, the compounds specified concretely as actual examples or examples in specifications of WO96/16080 and WO98/24806 are given as desirable compounds.

As more desirable compounds of them, compounds of the following (1) to (58) or these nontoxic salts are given.
Compound (1): 2-[5-amino-6-oxo-2-(4-fluorophenyl)-1,6-dihydro-1-pyrimidinyl]-N-[(1S)-1-[[5-(3-methylbenzyl)-1,3,4-oxadiazole-2-yl]carbonyl]-2-methylpropyl]acetamide (CAS Registry No. 208840-39-5) 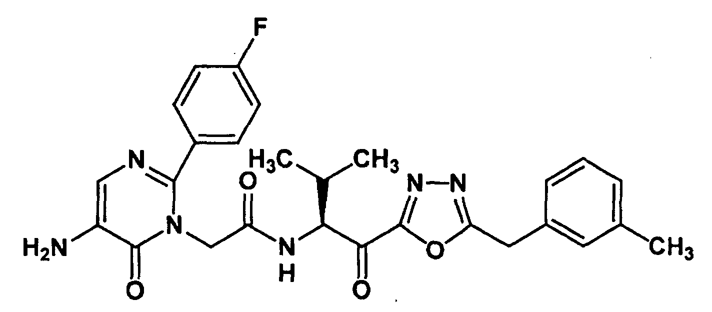
Compound (2): (2S,5S)-4-oxo-amino-5-1,2,4,5,6,7-hexahydro-N-[(1S)-1-[[5- 3-methylbenzyl)-1,3,4-oxadiazole-2-yl]carbonyl]-2-methylpropyl]azepino-[3,2,1]-indole-2-carboxamide (CAS Registry No. 208845-71-0) , 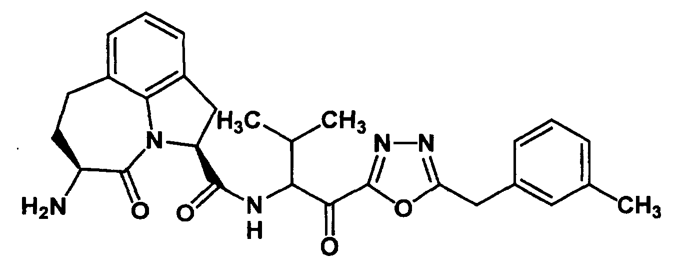
Compound (3): 2-[3[amino-2-oxo-5-phenyl-1,4-benzodiazepinyl]-N-1-[[5-(3-methylbenzyl)-1,3,4-oxadiazole-2-yl]carbonyl]-2-methylpropyl]acetamide (CAS Registry No. 208845-74-3) 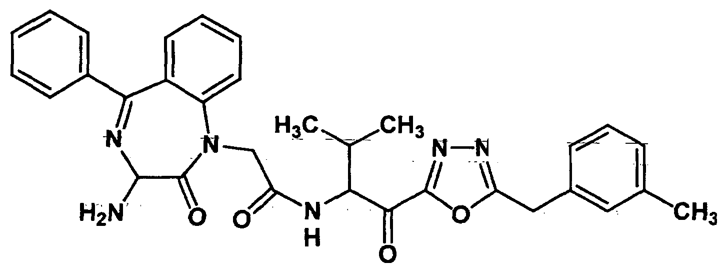
Compound (4): Methylsulfonyl-L-valyl-N-[(1S)-1-[[5-(3-methylbenzyl)-1,3,4-oxadiazole-2-yl]carbonyl]-2-methylpropyl]-L-prolinamide (CAS Registry No. 208846-01-9) , 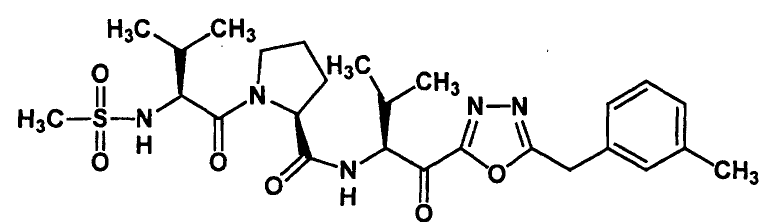
Compound (5): 2-[4-(R)- sopropyl-2,5-imidazolidinedione-1-yl]-N-[(1S)-1-[[5-(3-methylbenzyl)-1,3,4-oxadiazole-2-yl]carbonyl]-2-methylpropyl]acetamide (CAS Registry No. 208846-10-0), 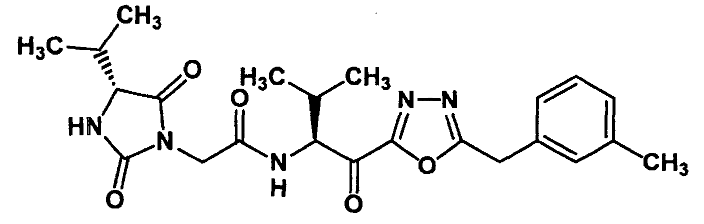
Compound (6): Benzyloxycarbonyl-L-valyl-N-[(1S)-1-[[5-(3-methylbenzyl)-1,3,4-oxadiazole-2-yl]carbonyl]-2-methylpropyl]-L-prolinamide (CAS Registry No. 208846-12-2), 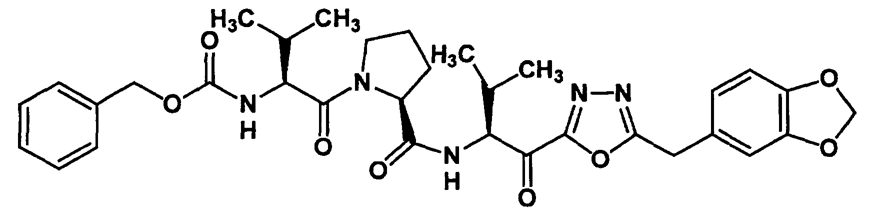
Compound (7): 2-[3-(2-(morpholine-2-yl)ethyl)-4-phenyl-2,5- imidazolidinedione-1-yl]-N-[1-[[5-(3-methylbenzyl)-1,3,4-oxadiazole-2-yl]carbonyl]-2-methylpropyl] acetamide (CAS Registry No. 208846-26-8), 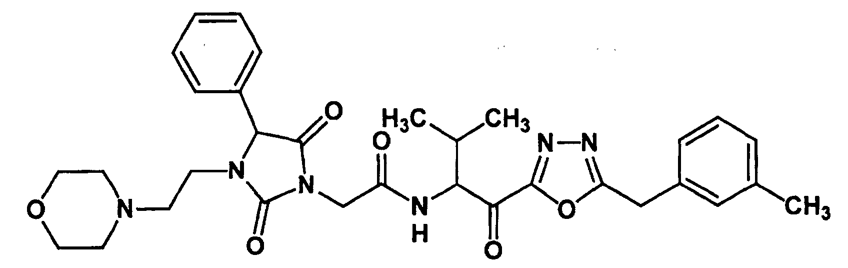
Compound (8): 2-[4-methyl-4-(pyridine-2-yl)-2,5-imidazolidinedione-1-yl]-N-1-[[5-(3-methylbenzyl)-1,3,4-oxadiazole-2-yl]carbonyl]-2-methylpropyl] acetamide (CAS Registry No. 208846-58-6), 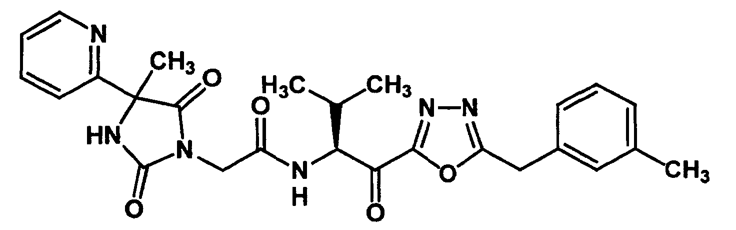
Compound (9): 2-[(4S)-4-(2-methylpropyl)-2,5-imidazolidinedione-1-yl]-N-1-[[5-(3-methylbenzyl)-1,3,4-oxadiazole-2-yl]carbonyl]-2-methylpropyl]acetamide (CAS Registry No. 208846-86-0), 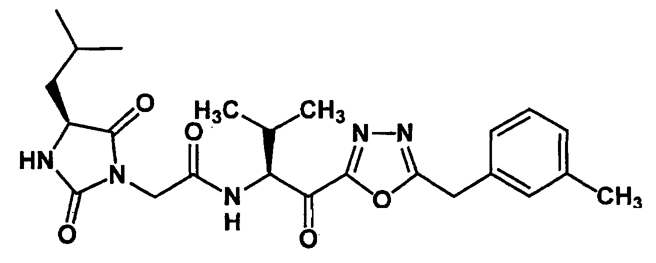
Compound (10): 2-[5-amino-6-oxo-2-(4-fluorophenyl)-1,6-dihydro-1-pyrimidinyl]-N-[(1S)-1-[[5-(α,α-methylbenzyl)-1,3,4-oxadiazole-2-yl]carbonyl]-2-methylpropyl]acetamide (CAS Registry No. 208840-37-3), 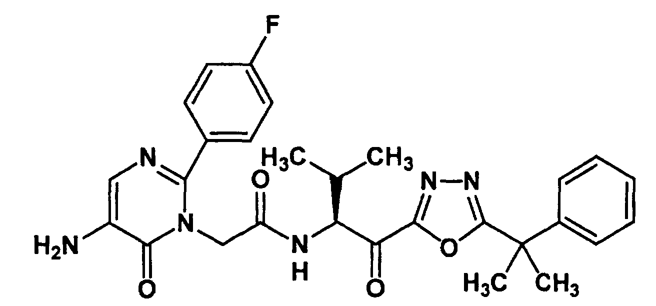
Compound (11): 2-[5-amino-6-oxo-2-(4-fluorophenyl)-1,6-dihydro-1-pyrimidinyl]-N-[(1S)-1-[[5-(α,α-dimethyl-3-methylbenzyl)-1,3,4-oxadiazole-2-yl]carbonyl]-2-methylpropyl]acetamide (CAS Registry No. 208840-38-4), 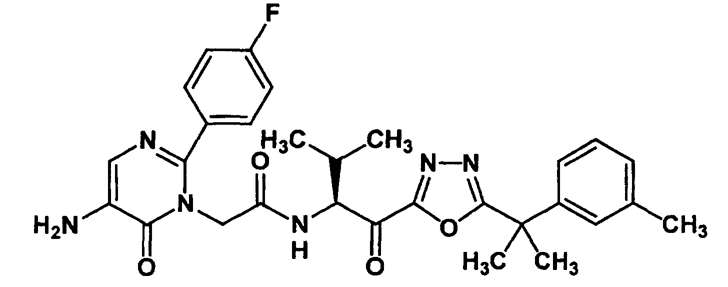
Compound (12): 2-[5-amino-6-oxo-2-(4-fluorophenyl)-1,6-dihydro-1-pyrimidinyl]-N-[(1R)-1-[[5-(3-methylbenzyl)-1,3,4-oxadiazole-2-yl]carbonyl]-2-methylpropyl]acetamide (CAS Registry No. 251988-66-6), 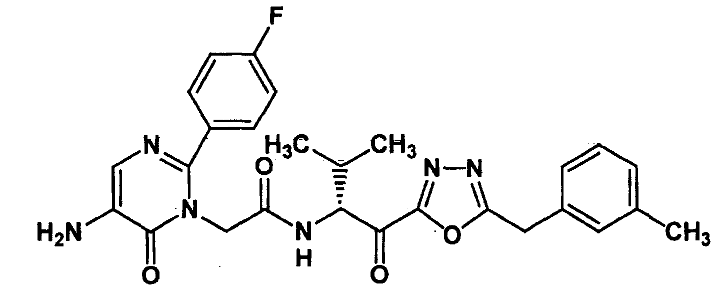
Compound (13): 2-[5-amino-6-oxo-2-(4-fluorophenyl)-1,6-dihydro-1-pyrimidinyl]-N-[(1S)-1-[[5-phenyl-1,3,4-oxadiazole-2-yl]carbonyl]-2-methylpropyl]acetamide (CAS Registry No. 208847-78-3), 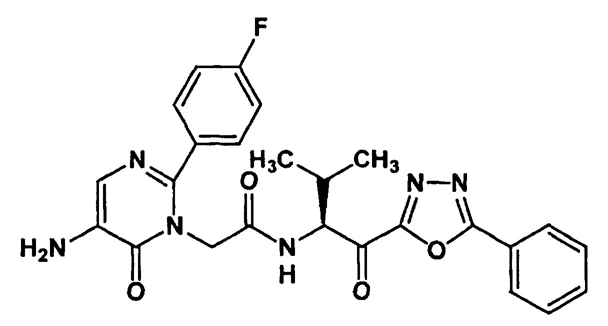
Compound (14): 2-[5-amino-6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208847-80-7). 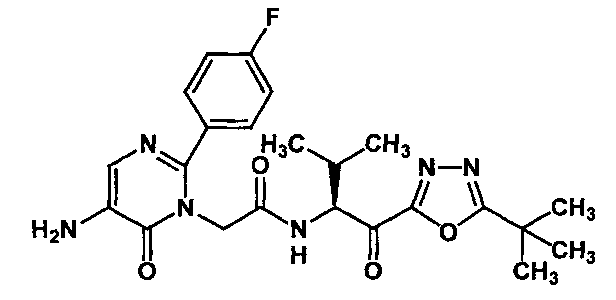
Compound (15): 2-[5-amino-6-oxo-2-(4-fluorophenyl)-1,6-dihydro-1-pyrimidinyl]-N-[(1S)-1-[[5-(α,α-dimethyl-3,4-methylenedioxybenzyl)-1,3,4-oxadiazole-2-yl]carbonyl]-2-methylpropyl]acetamide (CAS Registry No. 208847-84-1), 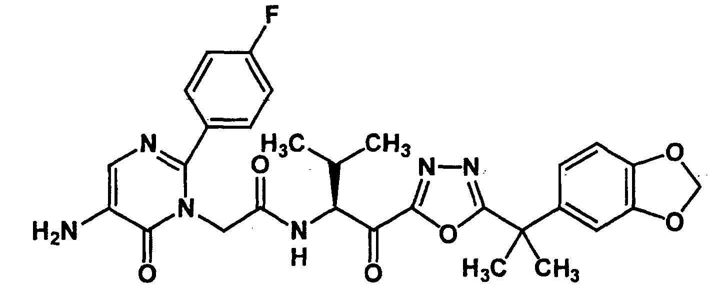
Compound (16): 2-[5-amino-6-oxo-2-(4-fluorophenyl)-1,6-dihydro-1-pyrimidinyl]-N-[(1S)-1-[[5-benzyl-1,3,4-oxadiazole-2-yl]carbonyl]-2-methylpropyl]acetamide (CAS Registry No. 208847-87-4), 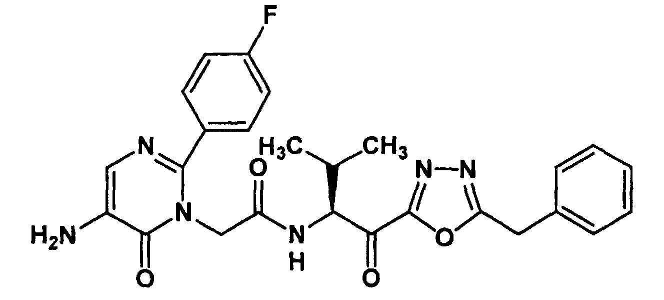
Compound (17): 2-[5-amino-6-oxo-2-(4-fluorophenyl)-1,6-dihydro-1-pyrimidinyl]-N-[(1S)-1-[[5-methyl-1,3,4-oxadiazole-2-yl]carbonyl]-2-methylpropyl]acetamide (CAS Registry No. 208847-88-5), 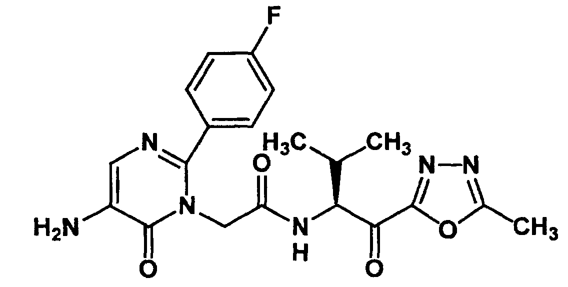
Compound (18): 2-[5-amino-6-oxo-2-(4-fluorophenyl)-1,6-dihydro-1-pyrimidinyl]-N-[(1S)-1-[[5-(1-methylethyl)-1,3,4-oxadiazole-2-yl]carbonyl]-2-methylpropyl]acetamide (CAS Registry No. 208847-89-6), 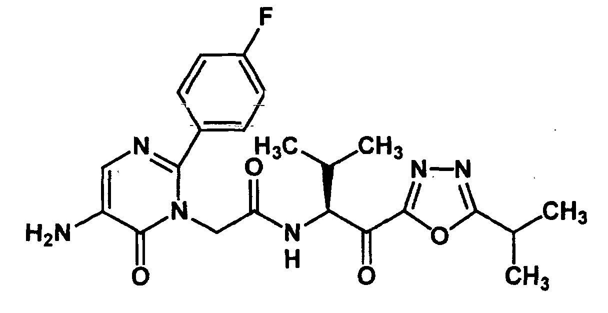
Compound (19): 2-[5-amino-6-oxo-2-(4-fluorophenyl)-1,6-dihydro-1-pyrimidinyl]-N-[(1S)-1-[[5-butyl-1,3,4-oxadiazole-2-yl]carbonyl]-2-methylpropyl]acetamide (CAS Registry No. 208847-90-9), 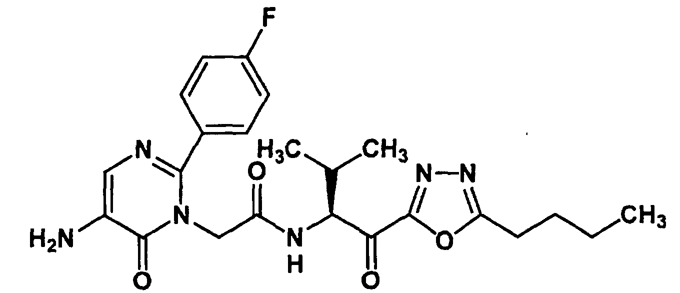
Compound (20): 2-[5-amino-6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208847-91-0), 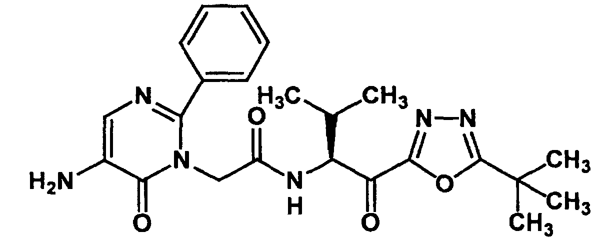
Compound (21): 2-[5-amino-6-oxo-2-phenyl-1,6-dihydro-1-pyrimidinyl]-N-[(1S)-1 -[[5-(α,α-dimethylbenzyl)-1,3,4-oxadiazole-2-yl]carbonyl]-2-methylpropyl]acetamide (CAS Registry No. 208847-95-4), 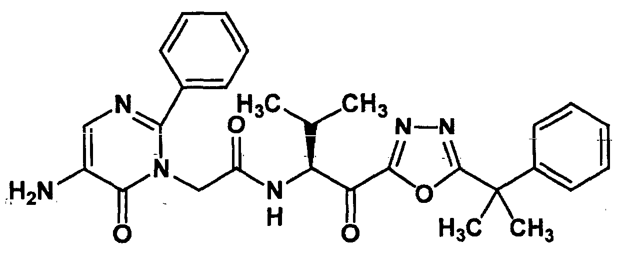
Compound (22): 2-[5-amino-6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208847-98-7), 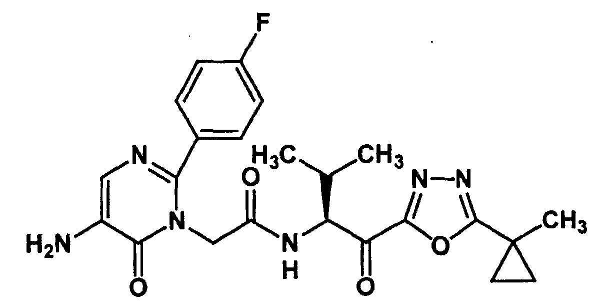
Compound (23): 2-[6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208848-00-4), 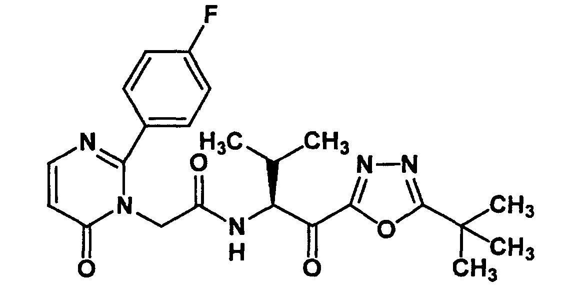
Compound (24): 2-[5-amino-6-oxo-2-(4-fluorophenyl)-1,6-dihydro-1-pyrimidinyl]-N-[( 1R)-1-[[5-(t-butyl)-1,3,4-oxadiazole-2-yl]carbonyl]-2-methylpropyl]acetamide (CAS Registry No. 208848-02-6), 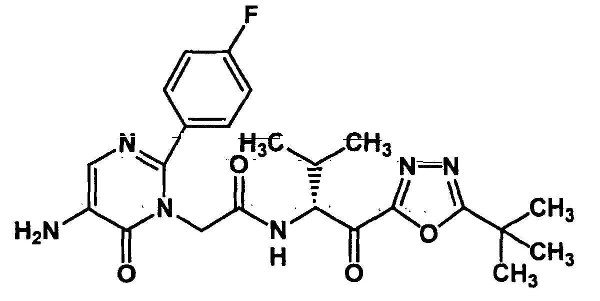
Compound (25): 2-[5-amino-6-oxo-2-(4-fluorophenyl)-1,6-dihydro-1-pyrimidinyl]-N-[(1R)-1-[[5-(α,α-dimethylbenzyl)-1,3,4-oxadiazole-2-yl]carbonyl]-2-methylpropyl]acetamide (CAS Registry No. 208848-05-9), 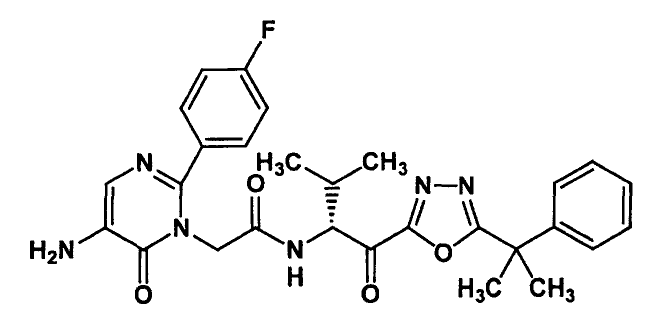
Compound (26): 2-[6-oxo-2-(4-fluorophenyl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208848-06-0), 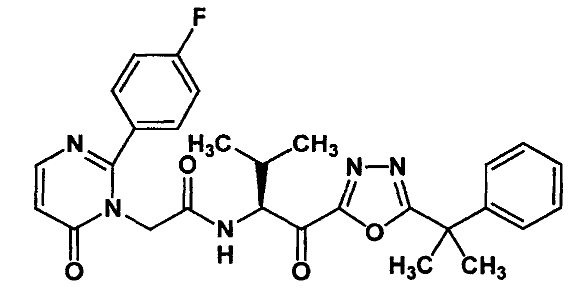
Compound (27): 2-[5-amino-6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-12-8), 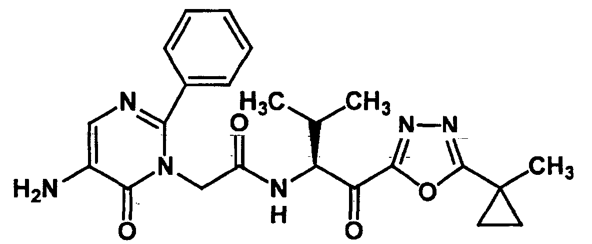
Compound (28): 2-[5-amino-6-oxo-2-phenyl-1,6-dihydro-1-pyrimidinyl]-N-[(1 R)-1-[[5-(t-butyl)-1,3,4-oxadiazole-2-yl]carbonyl]-2-methylpropyl]acetamide (CAS Registry No. 208848-13-9), 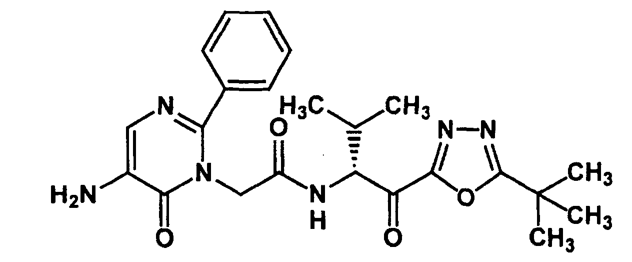
Compound (29): 2-[6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208848-14-0), 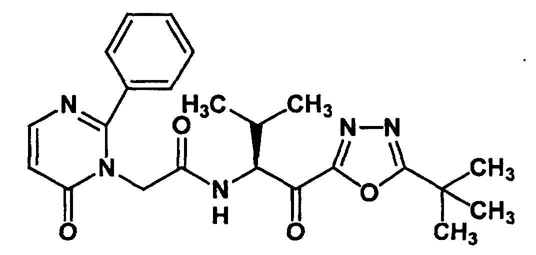
Compound (30): 2-[6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide (CAS Registry No. 208848-09-3), 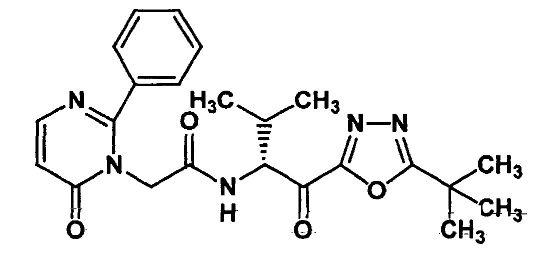
Compound (31): 2-[5-amino-6-oxo-2-(4-fluorophenyl)-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-16-2), 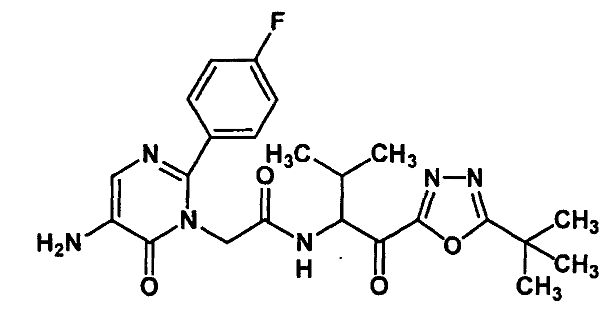
Compound (32): 2-[6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-17-3). 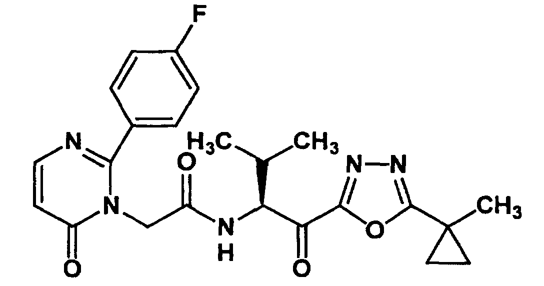
Compound (33): 2-[6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide (CAS Registry No. 208848-18-4), 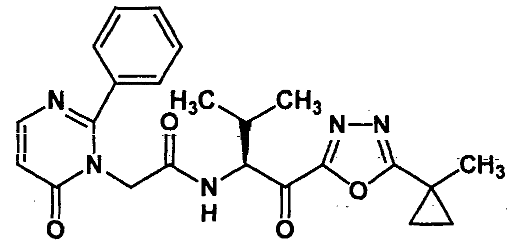
Compound (34): 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-19-5), 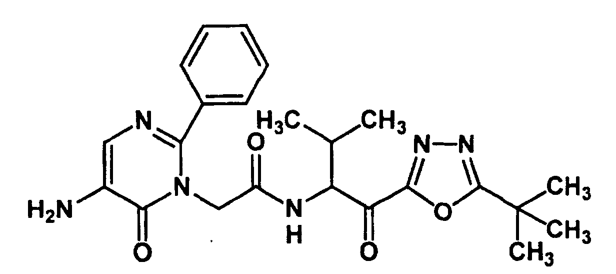
Compound (35): 2-[6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 208848-20-8), 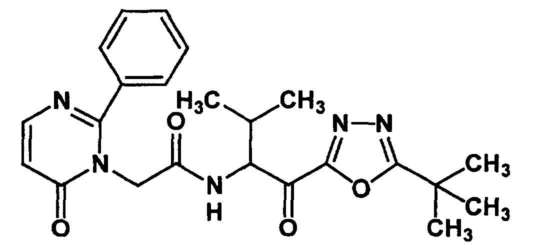
Compound (36): 2-[5-amino-6-oxo-2-(pyridine-3-yl)-1,6-dihydro-1-pyrimidinyl]-N-[(1S)-1-[[5-(t-butyl)-1,3,4-oxadiazole-2-yl]carbonyl]-2-methylpropyl]acetamide (CAS Registry No. 208847-96-5). 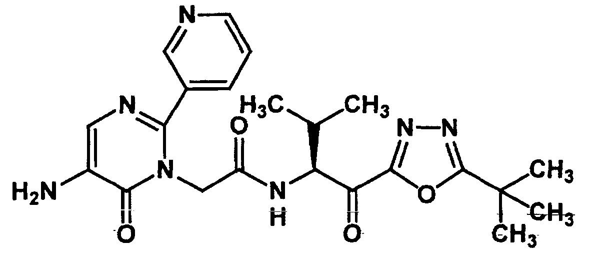
Compound (37): 2-[5-amino-6-oxo-2-(pyridine-3-yl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208847-99-8), 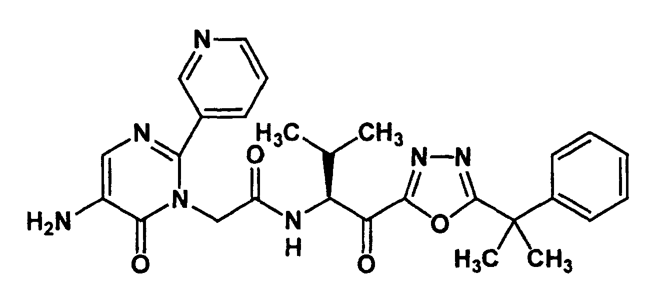
Compound (38): 2-[5-amino-6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-28-8), 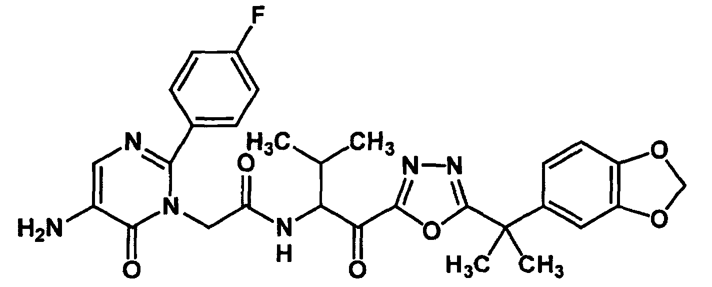
Compound (39): 2-[5-amino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-31-3), 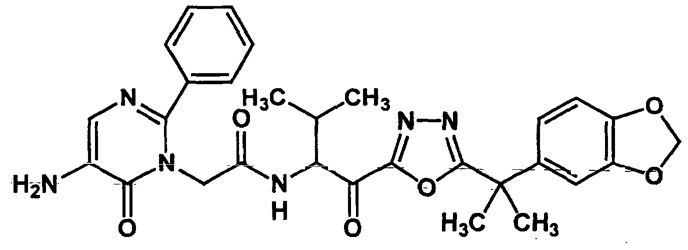
Compound (40): 2-[6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (CAS Registry No. 251958-33-5), 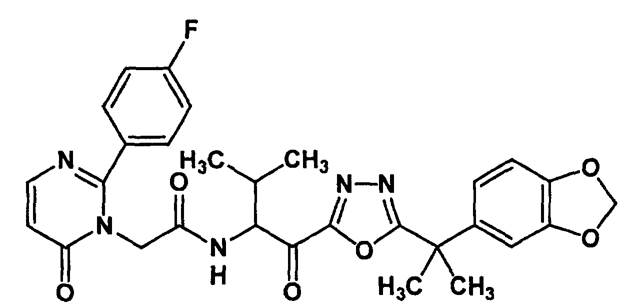
Compound (41): 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-32-4), 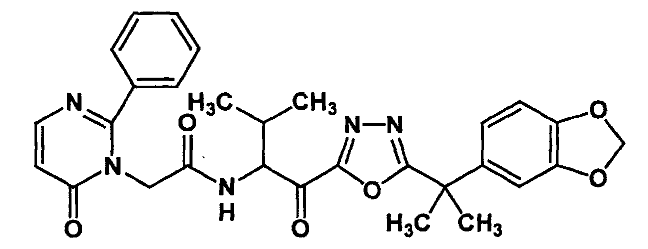
Compound (42): 2-[6-oxo-2-(4-fluorophenyl)-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-34-6), 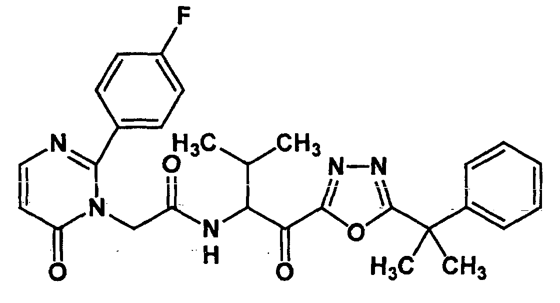
Compound (43): 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide (CAS Registry No. 251958-35-7), 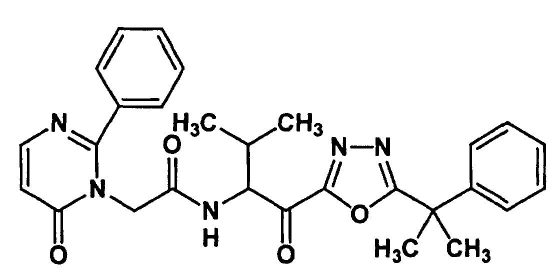
Compound (44): Methoxycarbonyl-L-valyl-N-[(1S)-1-[[5-(t-butyl)-1,3,4-oxadiazole-2-yl]carbonyl]-2-methylpropyl]-L-prolinamide (CAS Registry No. 251958-25-5). 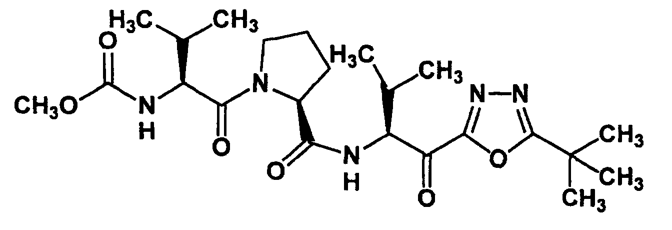
Compound (45): Methoxycarbonyl-L-valyl-N-[(1S)-1-[[5-(α,α-dimethylbenzyl)-1,3,4-oxadiazole-2-yl]carbonyl]-2-methylpropyl]-L-prolinamide (CAS Registry No. 251958-26-6) , 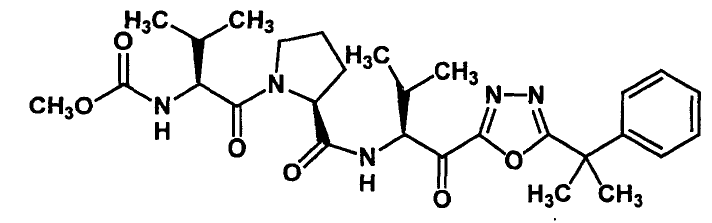
Compound (46): Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide (CAS Registry No. 251958-27-7), 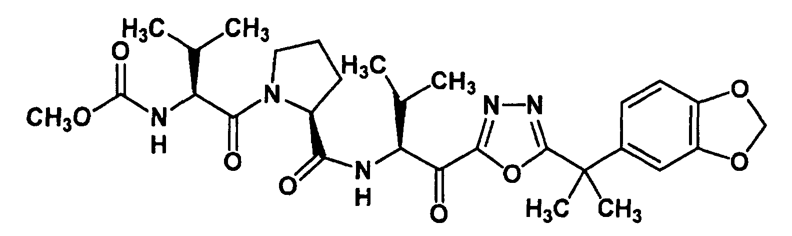
Compound (47): 2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (CAS Registry No. 251958-36-8), 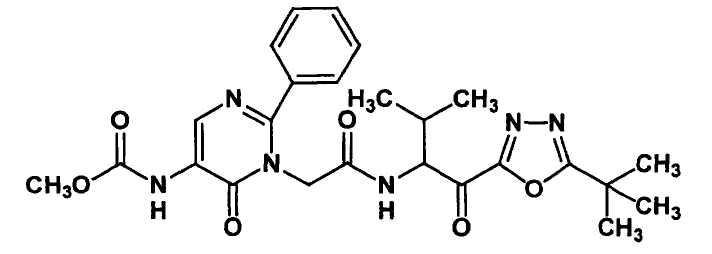
Compound (48): 1-[(3S)-(2-(t-butoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-43-9), 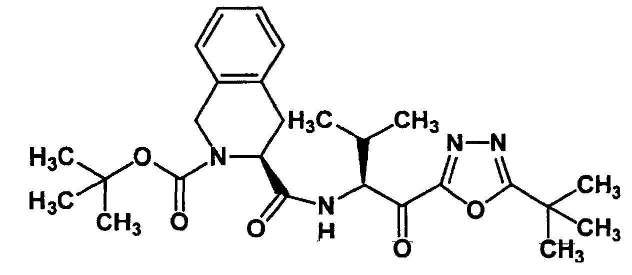
Compound (49): 1-[(3S)-(2-(1-methylethoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-44-0), 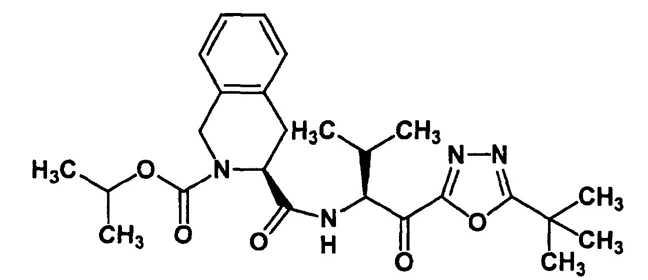
Compound (50): 1-[(3S)-(2-(imidazole-5-ylcarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-45-1), 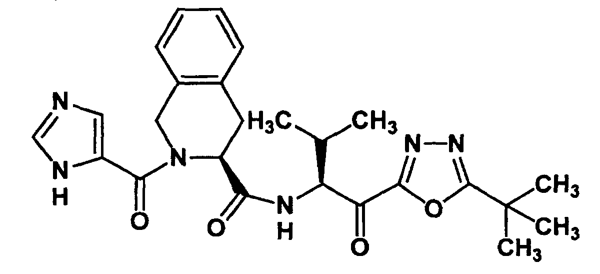
Compound (51): 1-[(3S)-(2-[(1S)-1-(methoxycarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-46-2), 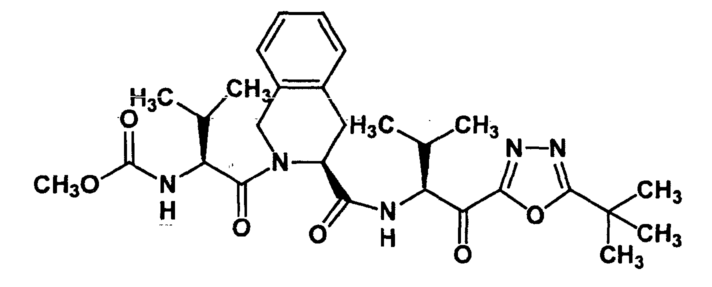
Compound (52): 1-[(3S)-(2-[(1S)-1-(pyridine-3-ylcarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-47-3), 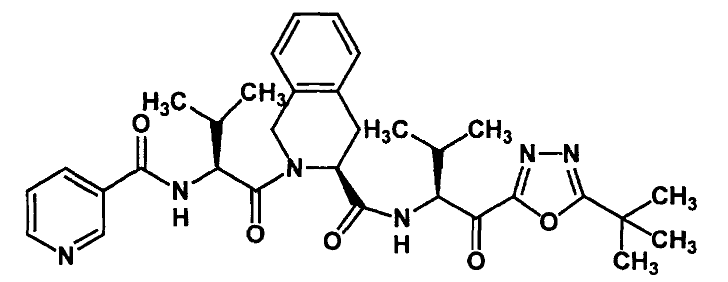
Compound (53): 1-[(2S)-1-(t-butoxycarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-48-4), 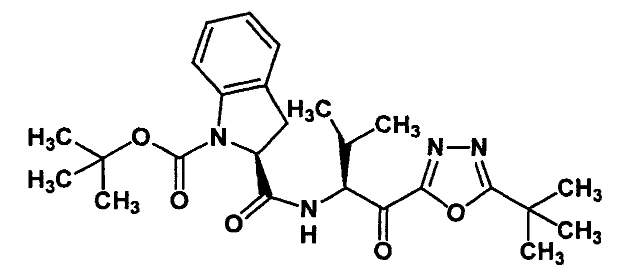
Compound (54): 1-[(2S)-1-(2-methylethoxycarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-49-5), 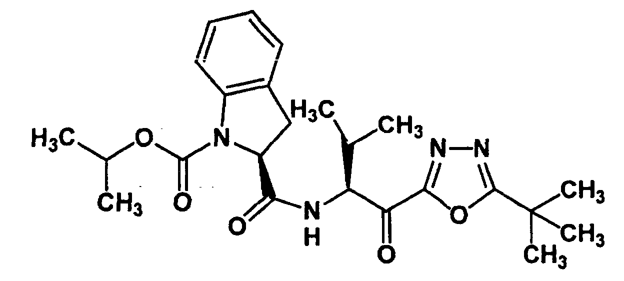
Compound (55): 1-[(2S)-1-(imidazole-5-ylcarbonyl)-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-50-8), 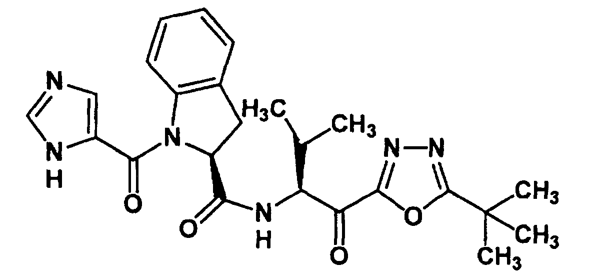
Compound (56): 1-[(2S)-1-[(1S)-1-(methoxycarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-51-9), 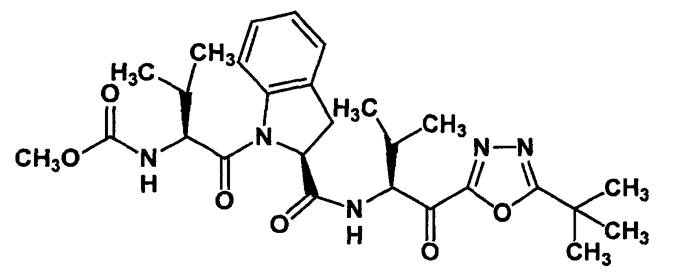
Compound (57): 1-[(2S)-1-[(1S)-1-(pyridine-3-ylcarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide (CAS Registry No. 251540-52-0) or 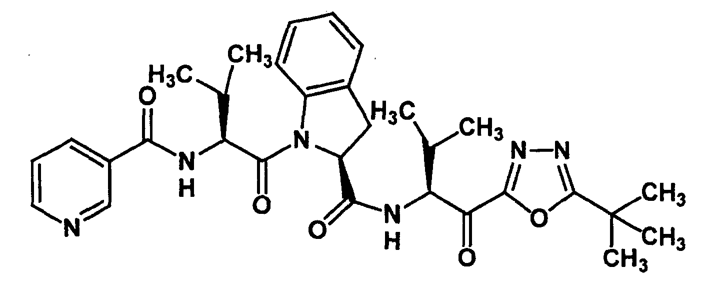
Compound (58): 2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide.

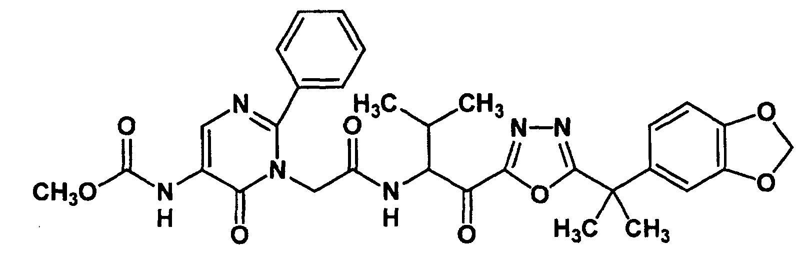

More desirable compounds are
2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (compound 34),
Methoxycarbonyl-L-valyl-N-[(1S)-1-[[5-(t-butyl)-1,3,4-oxadiazole-2-yl]carbonyl]-2-methylpropyl]-L-prolinamide (compound 44),
Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide (compound 46),
2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (compound 47) or
2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide (compound 58).

The most desirable compound is 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (compound 34).

Compounds represented by the formula (I) used for the present invention may be used in the form of the nontoxicity salt allowed in pharmacology. As for these salts, the ones of the soluble without toxicity are desirable.

As a suitable salt, the inorganic acid salt such as hydrochloride, hydrogen bromide salt, sulfate, phosphate salts or nitrates, or the organic acid salt such as acetates, trifluoroacetates, lactic acid salts, tartrate salts, malates, oxalates, fumarates, maleates, citrates, benzoate salts, methanesulfonates, the ethane sulfonic acid salts, benzenesulfonates, toluenesulfonates, isethionic acid salts, glucuronate salts or gluconates are enumerated. Moreover, compounds represented by the formula (I) or the salts can be converted into the hydrate by a well-known method.

In the present invention, isomers include all isomers as long as it does not especially direct it. For instance, the one of the straight chain and the one of the branched-chain are included in alkyl, alkenyl, alkynyl group and the alkylene. In addition, all isomers (E, Z, cis and transformer body) in double bond, ring, condensed ring, isomer (R, S body, α, β body, enantiomer, and diastereomer) by existence of asymmetric carbons etc., the optical isomer (D, L, d, and I body) with optical rotation, polarity body (high polarity body and low polarity body) separated by the chromatographic, balanced compounds, compounds of these arbitrary ratio and racemic mixtures are included in the present invention.

In addition, all the crystal forms where it can be taken are included in the compound shown by the formula (I) used by the present invention.

### BRIEF DESCRIPTION OF THE DRAWINGS

Figure 1 shows a graph to suggest the relation between the dosage of compound 34 and the area of ulcer in the acetate-caused colitis model experiment.
Figure 2 shows a graph to suggest the relation between the dosage of compound 34 and the amount of haemoglobin in the acetate-caused colitis model experiment.
Figure 3 shows a graph to suggest the relation between the dosage of compound 34 and the area of ulcer in the picrylsulfonic acid(TNBS)-caused colitis model experiment.
Figure 4 shows a graph to suggest the relation between the dosage of compound 34 and the amount of haemoglobin in the picrylsulfonic acid(TNBS)-caused colitis model experiment.

### THE BEST FORM TO EXECUTE INVENTION

The suppressive effect of the present invention compounds on ulcerative colitis was proven by the following experiments, however, this invention is not limited to them.

### (1) Acetate-caused colitis model.

After 1% aqueous acetic acid (10ml/kg) is injected from anus of Syrian strain male hamster (7 weeks of ages) into large intestine with a flexible, oral sonde for rat, colitis was caused by clipping anus for 30 minutes. Distilled water was similarly injected into a normal group.

0.5% CMC (10ml/kg) was orally administered to the normal group and the control group, and 10, 30 and 100mg/10ml/kg of a test drug suspended into 0.5% CMC were orally administered to the group administrated test drug three times in total, 18 hours and 1 hour before cause of colitis and 6 hours after cause, respectively.

They were anatomized 24 hours later, and large intestines of 7cm length were sampled from anus. Then, these intestinals were incised, and these enterics were washed by physiological salt solution (5ml). they were taken a picture of incised large intestines, and ulcer area (cm²) was calculated by the image analysis. Moreover, the washing supernatant of large intestine was supplied for the measurement of the amount of the haemoglobin (index of the amount of the enteric hemorrhage).

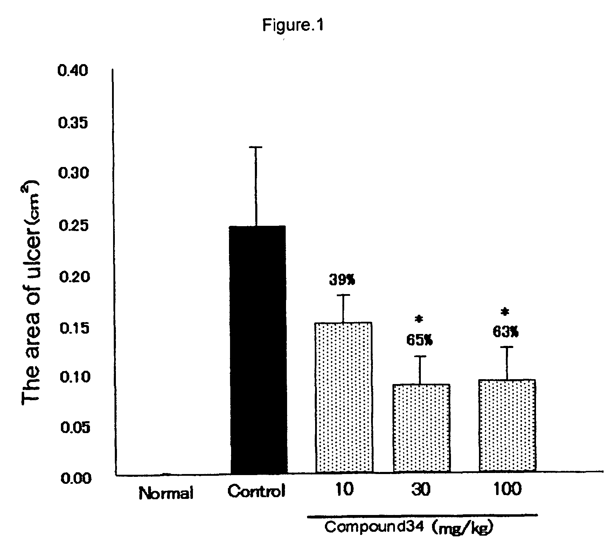

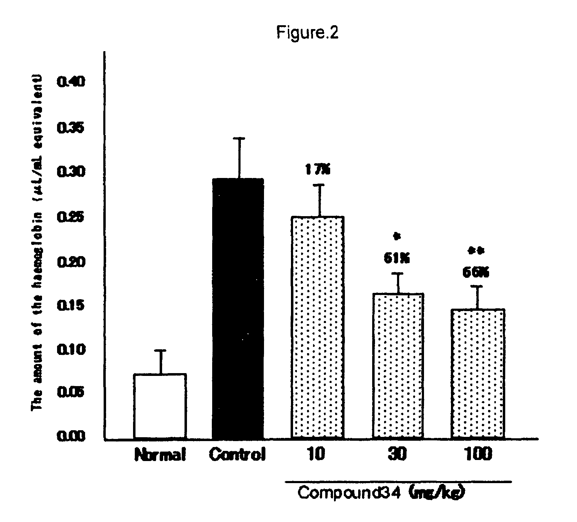

As a test drug, 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide (Compound 34) was used. Figure 1 and figure 2 show the results.

These results showed that the area of ulcer and the amount of the haemoglobin that was the index of the enteric hemorrhage were intentionally suppressed by administering 30 and 100 mg/kg of compound 34.

### (2)Picrylsulfonic acid(TNBS)-caused colitis model.

After 90mg/ml of TNBS/isotonic solution (with 15vol% ethanol) liquor (0.1ml/body) is injected from anus of Syrian strain male hamster (7 weeks of ages) intolarge intestine with a flexible, oral sonde for rat, colitis was caused by clipping anus for 30 minutes. Distilled water was similarly injected into a normal group.

0.5% CMC (10ml/kg) was orally administered to the normal group and the control group, and 25, 50 and 100mg/10ml/kg doses of a test drug suspended into 0.5% CMC were orally administered to the group administrated test drug once a day before cause of colitis and twice a day until the day of cause before anatomy, respectively.

They were anatomized 5 days later, and large intestines of 3cm length were sampled from anus. Then, these intestinals were incised, and these enterics were washed by physiological salt solution (2ml). they were taken a picture of incised large intestines, and ulcer area (cm²) was calculated by the image analysis. Moreover, the supernatant of washing of large intestine was supplied to the measurement of the amount of the haemoglobin (index of the amount of the enteric hemorrhage).

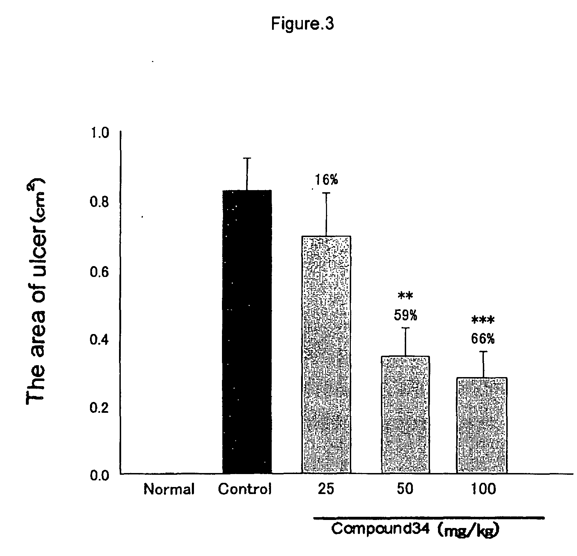

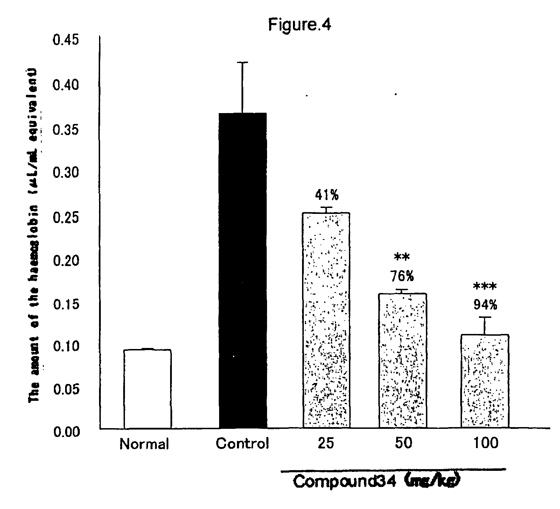

As a test drug, compound 34 was used as well as example (1). Figure 3 and figure 4 show the results.

These results showed that an area of the ulcer and the amount of the haemoglobin that was the index of the enteric hemorrhage were intentionally suppressed by administering 50 and 100 mg/kg of compound 34.

### INDUSTRIAL APPLICABILITY

Because compound 34 intentionally decreases the area of the ulcer in two typical models acknowledged as a model of ulcerative colitis, it was understood that compounds of the present invention could have high effectiveness. In addition, it was understood to intentionally suppress the enteric hemorrhage, significantly decreasing the amount of the haemoglobin. At this time, these facts were confirmed at the first time.

Therefore, it could be judged that five-member compounds represented by the formula (I) are effective in ulcerative colitis.

### [Toxicity]

It could be judged that the toxicity of compounds represented by the formula (I) used for the present invention are very low, and they are very safe to use them as a medicine.

### [Application to medicine]

The compounds shown by the formula (I) used for the present invention are useful in therapy for ulcerative colitis, for instance, ulcerative colitis, Crohn's disease, the ischemic colitis and the intestinal Behcet's syndrome, etc. in the animal including human, especially in human.

To use compounds of the present invention, the nontoxicity salts or the hydrates for the above purpose, they are usually administered as the form of the oral or non-oral systemically or locally.

A dosage is different depending on age, weight, symptom, therapeutic effect, administration mode, and processing time, etc., and they are orally administered once or several times a day within the range of a dosage from 1mg to 1000mg for adult, or are parenterally administered (Intravenous administration, desirably) within the range of a dosage from 0.1mg to 100mg or are continually intravenously administered for 1 hour to 24 hours a day.

Because the dosage changes according to various conditions as the above, it might be enough in less amount than the above dosage or might be necessary beyond the pale.

When this compounds of the present invention are administered, they are used as solid medicines for taking for the oral administration, the liquid medicine for taking, injection drugs for parenteral administration, external preparations, inhalants, and suppositories, etc.

The tablet, the pill, the capsule, the powder, and the granule, etc. are included in the solid medicine for taking for the oral administration. A hard capsule and a soft capsule are included in the capsule.

One or more of activators in such the solid medicine for taking is/are itself/themselves or are mixed with vehicles (lactose, mannitol, glucose, microcrystalline cellulose, and starch, etc.), binders (hydroxypropylcellulose, polyvinylpyrrolidone, and magnesium aluminometasilicate, etc.), disintegrators (cellulose glycolic acid calcium etc.), glidants (magnesium stearate etc.), stabilizers or solubilizers (glutamate and aspartate, etc.), etc. and are used as making to pharmaceutical preparations according to the usual method. Moreover, if necessary, they may be coated with coating materials (saccharose, gelatin, hydroxypropylcellulose, and hydroxypropylmethylcellulose phthalate, etc.) or may be coated in the layer of two or more. In addition, capsules of materials absorbed like gelatin are included.

The liquid medicine for taking for the oral administration contains the solution, the suspension, the emulsion, the syrup drug, and the elixir, etc. allowed as medicines.

One or more of activators in such these liquid medicines is/are dissolved, suspended or emulsified-into-diluents-(purified water, ethanol or those mixing liquids, etc.) used generally. In addition, such this liquid medicine may contain penetrants, suspending agents, emulsifying agents, sweeteners, flavor medicines, aromatic substances, preservatives and buffers, etc.

Injections for parenteral administration include solid injection drugs used with being dissolved or being suspended into liquor, suspension, latex or time of use solvent. Injections are used with being dissolved, suspended or emulsified one or more of activators into solvent. As solvents, distilled water for injection, physiological salt solution, vegetable oil, propylene glycol, polyethylene glycol or alcohol group, etc. such as ethanols or the mixture is used. In addition, this injections may contain stabilizers, solubilizers (glutamate, aspartate, and polysorbate 80 (registered trademark), etc.), suspending agents, emulsifying agents, medicines of making to aponia, buffers or preservatives, etc. These are manufactured and prepared by sterilized or aseptic manipulation in the final process. Moreover, it is possible to use them by manufacturing the sterilized solid medicine such as freeze-drying goods, and then dissolving to distilled water for injection or other solvents made to the sterility or the sterility before use.

Other preparations for parenteral administration, which contain one or more of activators, include liquids for external use prescribed with usual methods, ointment drugs, coating drugs, inhalants, aerosols or pessarys for administering in suppository and vagina, etc.

Besides diluents generally used, aerosols may contain stabilizers like the sodium hydrogensulfite, buffers which gives isotonicity or isotonic medicines such as sodium chloride and, sodium citrate or citrates. The manufacturing method of aerosol has been described in detail, for example, in the U.S. patent No.2,868,691 and No.3,095,355.

### Example 1 of preparation

100 tablets that contained 50mg of the active constituent in a tablet, which are compressed after mix with the following each element by usual methods, were obtained.
· 2-[5-amino-6-oxo-2-(4-fluorophenyl)-1,6-dihydro-1-pyrimidinyl]-N-[1-(2-[5-tert-butyl-1,3,4-oxadiazole]carbonyl)-2-(R,S)-methylpropyl]acetamide 5.0g
· Carboxymethylcellulose calcium (disintegrator) 0.2g
· Magnesium stearate (lubricant) 0.1g
· Microcrystalline cellulose 4.7g

### Example 2 of preparation

100 ampoules which contained 20mg of the active constituent in a ampoule, which are sterilized by usual methods and be filled each 5ml to the ampoule followed by being freeze-dried by usual methods, were obtained.
· 2-[5-amino-6-oxo-2-(4-fluorophenyl)-1,6-dihydro-1-pyrimidinyl]-N-[1-(2-[5-tert-butyl-1,3,4-oxadiazole]carbonyl)-2-(R,S)-methylpropyl]acetamide 2.0g
· Mannitol 20g
· Distilled water 500ml

## Claims

1. Prophylactic and/or therapeutic drugs against ulcerative colitis, which include five-member heterocyclic compounds represented by the formula (I) or these nontoxic salts as active principle, 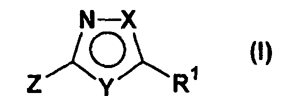 (wherein Z represents the group to contain α-aminocarbonyl group that carbonyl carbon atom combines with carbon atom of heterocyclic group through covalent bond,
R¹ represents (1) alkyl, alkenyl or alkynyl groups, with the proviso that these groups may be substituted with one or more groups chosen from (a)halogen atom, (b)hydroxy, (c)cyano, (d)nitro, (e)haloalkyl, (f)alkylamino, (g)dialkylamino (h)alkoxy, (l)haloalkoxy, (j)carboxy, (k)carboalkoxy, (l)alkylcaroxamide, (m)arylcaroxamide or (n)-O-(C5-C6) aryl groups,
(2)hydroxy,
(3)amino,
(4)alkylamino,
(5)dialkylamino, or
(6)cycloalkyl, alkylcycloalkyl, alkenylcycloalkyl, cycloalkenyl, alkylcycloalkenyl, alkenylcycloalkenyl, (C5-C12)aryl, (C5-C12)arylalkyl, (C5-C12)arylalkenyl, condensable (C5-C12)aryl-cycloalkyl or alkyl condensable (C5-C12)aryl-cycloalkyl, with the proviso that these groups may be substituted with groups chosen from (a)halogen atom, (b)hydroxy, (c)cyano, (d)nitro, (e)haloalkyl, (f)amino, (g)aminoalkyl, (h)dialkylamino, (i)alkyl, (j)alkenyl, (k)alkylenedioxy, (l)alkynyl, (m)alkoxy, (n)haloalkoxy, (o)carboxy, (p)carboalkoxy, (q)carboxamide, (r)(C5-C6)aryl, (s)-O-(C5-C6)aryl, (t)arylcarboxamide, (u)alkylthio or (v)haloaklylthio that may include 1-4 of hetero atoms chosen from nitrogen, oxygen or sulfur atom, respectively,
X and Y each independently represent oxygen, sulfur, nitrogen or atom,
which nitrogen atom may be substituted with groups chosen from
(1)alkyl or alkenyl that may be substituted-with 1-3 of halogen atom,
(2)alkynyl,
(3)(C5-C6)aryl, arylalkyl, arylalkenyl that may include 1-3 of hetero atoms chosen from nitrogen, oxygen or sulfur atom or may be substituted with groups chosen from (a)halogen atom, (b)cyano, (c)nitro, (b)hydroxy, (e)haloalkyl, (f)amino, (g)aminoalkyl, (h)dialkylamino, (i)alkyl, (j)alkenyl, (k)alkynyl, (l)alkoxy, (m)haloalkoxy, (n)carboxy, (o)carboalkoxy, (p)carboxamide, (q)arylcarboxamide, (r)alkylthio or (s)haloaklylthio).

2. Prophylactic and/or therapeutic drugs described in claim 1, wherein Z is represented by the formula (I-1), 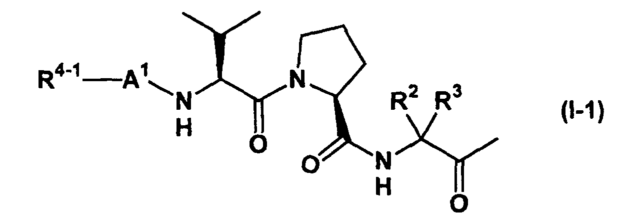 (wherein R² and R³ each independently represent alkyl, alkenyl, -RCOR', -RCOOR', -RNR'R"R^{o}, or -RC(O)NR'R" [wherein R represents alkyl or alkenyl, R', R" and R° each independently represent hydrogen atom, alkyl, alkenyl, cycloalkyl or (C5-C6)aryl.] with the proviso that the above groups optionally be substituted with hydrogen, 1-3 of halogen atom, hydroxy, sulfur atom, alkylthio, amino, alkylamino, dialkylamino, alkylguanidinyl, dialkylguanidinyl, guanidinyl or amidylguanidine; or cycloalkyl, alkylcycloalkyl, alkenylcycloalkyl, alkyl-oxyaryl, alkyl-thioaryl, alkyl-aminoaryl, (C5-C12)aryl, (C5-C12)arylalkyl or (C5-C12)arylalkenyl with the proviso that the above groups optionally include 1-4 of hetero atoms chosen from nitrogen, oxygen or sulfur atom and are substituted with halogen atoms, cyano, keto, nitro, hydroxy, haloalkyl, amino, aminoalkyl, dialkylamino, amidine, alkylamidine, dialkylamidine, alkyl, alkenyl, alkylenedioxy, alkynyl, alkoxy, haloalkoxy, carboxy, carboalkoxy, alkylcarboxamide, (C5-C6)aryl, -O-(C5-C6)aryl, arylcarboxamide, alkylthio or haloalkylthio,
A¹ represents direct coupling, -C(O)-, -NH-C(O)-, -S(O)₂-, -NH-S(O)₂-, -C(O)-, -C- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom,
R⁴⁻¹ represents cycloalkyl, alkylcycloalkyl, (C5-C12)aryl, (C5-C12)arylalkyl, condensed (C5-12)aryl-cycloalkyl or condensed alkyl(C5-C12)aryl-cycloalkyl with the proviso that these groups optionally include hydrogen atom, alkyl, alkenyl or alkynyl, one or more hetero atoms chosen from nitrogen, oxygen or sulfur atom, and optionally be substituted with alkyl, alkenyl, alkynyl, halogen atom, cyano, nitro, hydroxy, haloalkyl, alkoxy, amino, aminoalkyl, dialkylamino, carboxy, haloalkoxy, carboalkoxy, alkylcarboxamide, aryl, arylalkyl, arylcarboxamide, alkylthio or haloalkylthio).

3. Prophylactic and/or therapeutic drugs described in claim 1, wherein Z is represented by the formula (1-2) 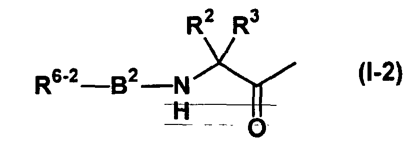 (wherein R² and R³ represent the same meanings as aforesaid;
B² represents -S(O)₂-, -C(O)-, -OC(O)- or -CH₂C(O)-;
R⁶⁻² represents the following groups; 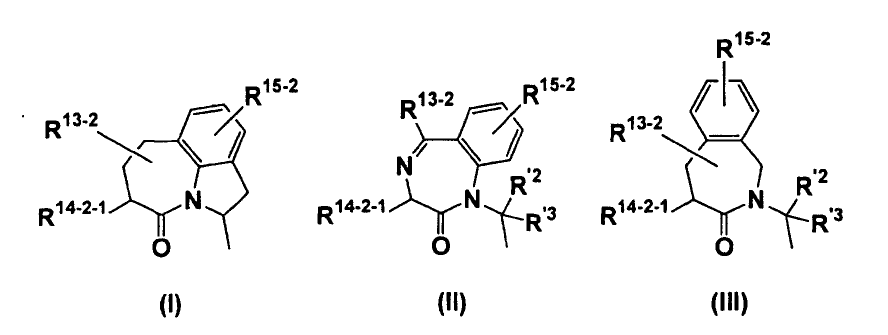 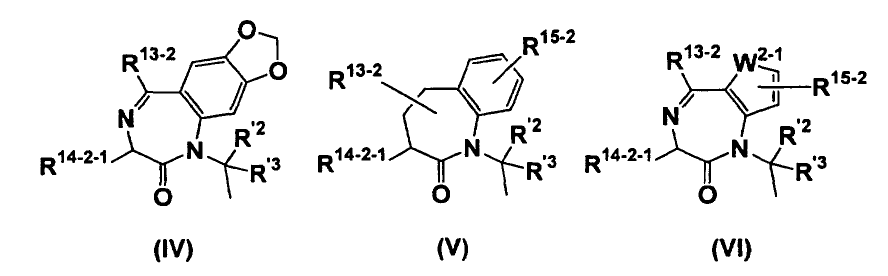 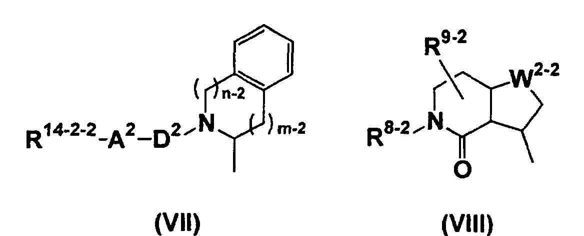 (wherein R'² and R'³ represent the same meanings as R² and R³;
R¹³⁻² represents aryl, arylalkyl, cycloalkyl, alkylcycloalkyl, condensed aryl-cycloalkyl, alkyl condensed aryl-cycloalkyl with the proviso that these groups optionally include hydrogen atom, alkyl, halogen atom, alkoxy, carboalkoxy, carboxy, alkylthio, amino, alkylamino, dialkylamino or one or more hetero atoms chosen from nitrogen, oxygen or sulfur-atom,-and-optionally be substituted with halogen atom or alkyl;
R¹⁴⁻²⁻¹ represents aryl, arylalkyl, cycloalkyl, alkylcycloalkyl, condensed aryl-cycloalkyl, alkyl condensed aryl-cycloalkyl or aryloxycarboxamide with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, arylalkylcarboxamide, alkylthio or haloalkylthio;
R¹⁵⁻² represents aryl, arylalkyl, cycloalkyl, alkylcycloalkyl, condensed aryl-cycloalkyl or alkyl condensed aryl-cycloalkyl with the proviso that these groups optionally include halogen atom, alkyl, halogen atom, alkoxy, carboalkoxy, carboxy, alkylthio, amino, alkylamino, dialkylamino or one or more hetero atoms chosen from nitrogen, oxygen or sulfur atom;
W²⁻¹ represents oxygen or sulfur atom; carbon or nitrogen atom that optionally be substituted with hydrogen atom, alkyl or aryl;
[m-2] represents 0 or 1;
[n-2] represents 0 or 1;
D² represents direct coupling or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A² represents direct coupling, -C(O)-, -NH-C(O)-, -S(O)₂-, -NH-S(O)₂-, -C(O)- or -C-;
R¹⁴⁻²⁻² represents aryl, arylalkyl, cycloalkyl, alkylcycloalkyl, condensed aryl-cycloalkyl or alkyl condensed aryl-cycloalkyl with the proviso that these groups optionally include hydrogen atom, alkyl, alkenyl, amino, alkylamino, dialkylamino or one or more hetero atoms chosen from nitrogen, oxygen or sulfur atom, and optionally be substituted with alkyl, halogen atom, alkoxyl amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio;
W²⁻² represents sulfur or oxygen atom;
R⁸⁻² represents alkylamino dialkylamino or amino;
R⁹⁻² represents hydrogen atom, alkyl or halogen atom)).

4. Prophylactic and/or therapeutic drugs described in claim 1, wherein Z is represented by the formula (I-3), 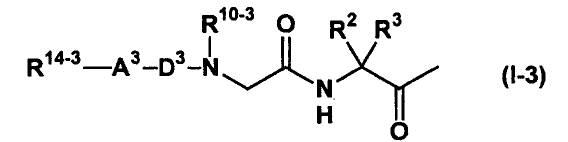 (wherein R² and R³ represent the same meanings as aforesaid,
R¹⁰⁻³ represents (C5-C6)aryl, (C5-C6)arylalkyl, (C5-C6)arylalkenyl, cycloalkyl, condensed aryl-cycloalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, alkylcarboxamide, alkylthio or haloalkylthio;
D³ represents direct coupling, -C(O)- or, for example, amino acids chosen from the following, amino acids aren't limited to these;
proline, isoleucine or cyclohexylalanine; cysteine, phenylalanine, homophenylalanine, dehydrophenylalanine, indoline-2-carboxylic acid with the proviso that the above amino acids optionally be substituted with alkyl, alkenyl or phenyl, and that sulfur atom optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthio; tetrahydroisoquinoline-2-carboxylic acid which optionally be substituted in alkyl, alkenyl or phenyl, with the proviso that these groups optionally be substituted with halogen atom, cyano, nitro, haloalkyl, amino, aminoalkyl, dialkylamino, alkyl, alkoxy, haloalkoxy, carboxy, carboalkoxy, alkylcarboxamide, arylcarboxamide, alkylthio or haloalkylthiotryptophan; tryptophane, tyrosine, serine, threonine; histidine, methionine, valine, norvaline, norleucine or octahydroindole-2-carboxylic acid with the proviso that these amino acids optionally be substituted with alkyl or aryl; asparagine, glutamine, ornithine or lysine with the proviso that nitrogen atoms in side chains of these amino acids optionally be substituted with alkyl, alkenyl, alkynyl, alkoxyalkyl, alkylthioalkyl, alkylaminoalkyl, dialkylaminoalkyl, carboxyalkyl, alkoxyalkyl, alkylthioalkyl, alkylaminoalkyl, dialkylaminoalkyl, carboxyalkyl, alkoxycarbonylalkyl, aryl, arylalkyl, cycloalkyl, alkylcycloalkyl, condensed aryl-cycloalkyl or alkyl condensed aryl-cycloalkyl and optionally include one or more hetero atoms chosen from nitrogen, oxygen or sulfur atom;
A³ represents direct coupling, -C(O)-, -NH-C(O)-, -S(O)₂-, -NH-S(O)₂-, -C(O)-, -S(O)2-NH-, OC(0)NH-, -OC(O)- or -C-;
R¹⁴⁻² and R¹⁴⁻²⁻¹ represent the same meanings as aforesaid).

5. Prophylactic and/or therapeutic drugs described in claim 1, wherein Z is represented by the formula (I-4), 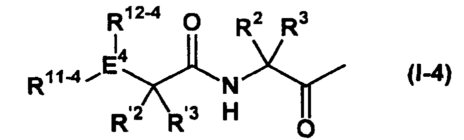 (wherein R², R³ R'² and R'³ represent the same meanings as aforesaid,
R¹¹⁻⁴, R¹²⁻⁴ and E⁴ are constituted of monocyclic or dicyclic ring with the proviso that these rings include 5-10 atoms chosen from carbon, nitrogen, sulfur or oxygen atom and include one or more keto groups and optionally be substituted with halogen atom, cyano, nitro, haloalkyl, amino, aminoalkyl, dialkylamino, alkyl, alkenyl, alkynyl, alkoxy, haloalkoxy, carboxy, carboalkoxy, alkylcarboxamide, alkylthio or haloalkylthio; and represent cycloalkyl, alkylcycloalkyl, alkenylcycloalkyl, (C5-C12)aryl, (C5-C12)arylalkyl, ((C5-C12)arylalkyl)OC(O)NH- or (C5-C12)arylalkyl with the proviso that these groups optionally include one or more hetero atoms chosen from nitrogen, sulfur or non-peroxideone oxygen atom, and optionally be substituted with halogen atom, cyano, nitro, haloalkyl, amino, aminoalkyl, dialkylamino, alkyl, alkenyl, alkoxy, haloalkoxy, carboxy, carboalkoxy, -C(O)O(alkyl), -C(O)(alkyl), alkylcarboxamide, alkylthio or haloalkylthio).

6. Prophylactic and/or therapeutic drugs described in claim 5, wherein R¹¹⁻⁴, R¹²⁻⁴ and E⁴ are represented by the formula (I-4), 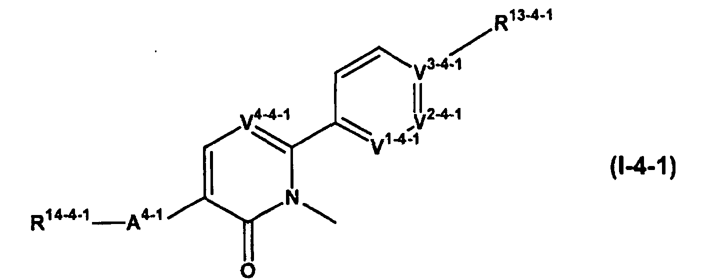 (wherein A⁴⁻¹ represents the same meanings as A³,
V¹⁻⁴⁻¹, V²⁻⁴⁻¹, V³⁻⁴⁻¹ and V⁴⁻⁴⁻¹ each independently represent carbon or nitrogen atom, in case V³⁻⁴⁻¹ represents carbon atom, R¹³⁻⁴⁻¹ represents hydrogen atom, alkyl, halogen atom, alkoxy, carboalkoxy, carboxy, alkylthio, amino, alkylamino, dialkylamino; aryl, arylalkyl, cycloalkyl, alkylcycloalkyl, condensed aryl-cycloalkyl or alkyl condensed aryl-cycloalkyl with the proviso that these groups optionally include one or more hetero atoms chosen from nitrogen, sulfur or oxygen atom, and optionally be substituted with halogen atom or alkyl;
R¹⁴⁻⁴⁻¹ represents hydrogen atom, alkyl, alkenyl, amino, alkylamino or dialkylamino; aryl, arylalkyl, cycloalkyl, alkylcycloalkyl, condensed aryl-cycloalkyl or alkyl condensed aryl-cycloalkyl, arylalkylcarbonyl or arylalkylcarboxamide with the proviso that these groups optionally include one or more hetero atoms chosen from nitrogen, sulfur or oxygen atom, and optionally be substituted with alkyl, halogen atom, alkoxy, amino, alkylamino, dialkylamino, carboxy, alkenyl, alkynyl, haloalkoxy, carboalkoxy, alkylcarboxamide, aryl, arylalkyl, arylcarboxamide, alkylthio or haloalkylthio).

7. Prophylactic and/or therapeutic drugs described in claim 1, wherein compounds are selected from;
(1) 2-[5-amino-6-oxo-2-(4-fluorophenyl)-1,6-dihydro-1-pyrimidinyl]-N-[(1S)-1-[[5-(3-methylbenzyl)-1,3,4-oxadiazole-2-yl]carbonyl]-2-methylpropyl]acetamide,
(2) (2S,5S)-4-oxo-amino-5-1,2,4,5,6,7-hexahydro-N-[(1S)-1-[[5- 3-methylbenzyl)-1,3,4-oxadiazole-2-yl]carbonyl]-2-methylpropyl]azepino-[3,2,1]-indole-2-carboxamide,
(3) 2-[3[amino-2-oxo-5-phenyl-1,4-benzodiazepinyl]-N-1-[[5-(3-methylbenzyl)-1,3,4-oxadiazole-2-yl]carbonyl]-2-methylpropyl]acetamide,
(4) Methylsulfonyl-L-valyl-N-[(1S)-1-[[5-(3-methylbenzyl)-1,3,4-oxadiazole-2-yl]carbonyl]-2-methylpropyl]-L-prolinamide,
(5) 2-[4-(R)- sopropyl-2,5-imidazolidinedione-1-yl]-N-[(1S)-1-[[5-(3-methylbenzyl)-1,3,4-oxadiazole-2-yl]carbonyl]-2-methylpropyl]acetamide,
(6) Benzyloxycarbonyl-L-valyl-N-[(1S)-1-[[5-(3-methylbenzyl)-1,3,4-oxadiazole-2-yl]carbonyl]-2-methylpropyl]-L-prolinamide,
(7) 2-[3-(2-(morpholine-2-yl)ethyl)-4-phenyl-2,5- imidazolidinedione-1-yl]-N-[1-[[5-(3-methylbenzyl)-1,3,4-oxadiazole-2-yl]carbonyl]-2-methylpropyl] acetamide,
(8) 2-[4-methyl-4-(pyridine-2-yl)-2,5-imidazolidinedione-1-yl]-N-1-[[5-(3-methylbenzyl)-1,3,4-oxadiazole-2-yl]carbonyl]-2-methylpropyl] acetamide,
(9) 2-[(4S)-4-(2-methylpropyl)-2,5-imidazolidinedione-1-yl]-N-1-[[5-(3-methylbenzyl)-1,3,4-oxadiazole-2-yl]carbonyl]-2-methylpropyl]acetamide,
(10) 2-[5-amino-6-oxo-2-(4-fluorophenyl)-1,6-dihydro-1-pyrimidinyl]-N-[(1S)-1-[[5-(α,α-methylbenzyl)-1,3,4-oxad iazole-2-yl]carbonyl]-2-methylpropyl]acetamide,
(11) 2-[5-amino-6-oxo-2-(4-fluorophenyl)-1,6-dihydro-1-pyrimidinyl]-N-[(1S)-1-[[5-(α,α-dimethyl-3-methylbenzyl)-1,3,4-oxadiazole-2-yl]carbonyl]-2-methylpropyl]acetamide,
(12) 2-[5-amino-6-oxo-2-(4-fluorophenyl)-1,6-dihydro-1-pyrimidinyl]-N-[(1R)-1-[[5-(3-methylbenzyl)-1,3,4-oxadiazole-2-yl]carbonyl]-2-methylpropyl]acetamide,
(13) 2-[5-amino-6-oxo-2-(4-fluorophenyl)-1,6-dihydro-1-pyrimidinyl]-N-[(1S)-1-[[5-phenyl-1,3,4-oxadiazole-2-yl]carbonyl]-2-methylpropyl]acetamide,
(14) 2-[5-amino-6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide,
(15) 2-[5-amino-6-oxo-2-(4-fluorophenyl)-1,6-dihydro-1-pyrimidinyl]-N-[(1S)-1-[[5-(α,α-dimethyl-3,4-methylenedioxybenzyl)-1,3,4-oxadiazole-2-yl]carbonyl]-2-methylpropyl]acetamide,
(16) 2-[5-amino-6-oxo-2-(4-fluorophenyl)-1,6-dihydro-1-pyrimidinyl]-N-[(1S)-1-[[5-benzyl-1,3,4-oxadiazole-2-yl]carbonyl]-2-methylpropyl]acetamide,
(17) 2-[5-amino-6-oxo-2-(4-fluorophenyl)-1,6-dihydro-1-pyrimidinyl]-N-[(1S)-1-[[5-methyl-1,3,4-oxadiazole-2-yl]carbonyl]-2-methylpropyl]acetamide,
(18) 2-[5-amino-6-oxo-2-(4-fluorophenyl)-1,6-dihydro-1-pyrimidinyl]-N-[(1S)-1-[[5-(1-methylethyl)-1,3,4-oxadiazole-2-yl]carbonyl]-2-methylpropyl]acetamide,
(19) 2-[5-amino-6-oxo-2-(4-fluorophenyl)-1,6-dihydro-1-pyrimidinyl]-N-[(1S)-1-[[5-butyl-1,3,4-oxadiazole-2-yl]carbonyl]-2-methylpropyl]acetamide,
(20) 2-[5-amino-6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide,
(21) 2-[5-amino-6-oxo-2-phenyl-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide (CAS Registry No. 208847-95-4),
(22) 2-[5-amino-6-oxo-2-(4-fluorophenyl)-1,6-dihydro-1-pyrimidinyl]-N-[(1S)-1-[[5-(1-methylcyclopropyl)-1,3,4-oxad iazole-2-yl]carbonyl]-2-methylpropyl]acetamide,
(23) 2-[6-oxo-2-(4-fluorophenyl)-1,6-dihydro-1-pyrimidinyl]-N-[(1S)-1-[[5-(t-butyl)-1,3,4-oxadiazole-2-yl]carbonyl]-2-methylpropyl]acetamide,
(24) 2-[5-amino-6-oxo-2-(4-fluorophenyl)-1,6-dihydro-1-pyrimidinyl]-N-[(1R)-1-[[5-(t-butyl)-1,3,4-oxadiazole-2-yl]carbonyl]-2-methylpropyl]acetamide,
(25) 2-[5-amino-6-oxo-2-(4-fluorophenyl)-1,6-dihydro-1-pyrimidinyl]-N-[(1R)-1-[[5-(α,α-dimethylbenzyl)-1,3,4-oxadiazole-2-yl]carbonyl]-2-methylpropyl]acetamide,
(26) 2-[6-oxo-2-(4-fluorophenyl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide,
(27) 2-[5-amino-6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(28) 2-[5-amino-6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide,
(29) 2-[6-oxo-2-phenyl-1,6-dihydro-1-pyrimidinyl]-N-[(1S)-1-[[5-(t-butyl)-1,3,4-oxadiazole-2-yl]carbonyl]-2-methylpropyl]acetamide,
(30) 2-[6-oxo-2-phenyl-1,6-dihydro-1-pyrimidinyl]-N-[(1R)-1-[[5-(t-butyl)-1,3,4-oxadiazole-2-yl]carbonyl]-2-methylpropyl]acetamide,
(31) 2-[5-amino-6-oxo-2-(4-fluorophenyl)-1,6-dihydro-1-pyrimidinyl]-N-[1-[[5-(t-bytyl)-1,3,4-oxadiazole-2-yl]carbonyl]-2-methylpropyl]acetamide,
(32) 2-[6-oxo-2-(4-fluorophenyl)-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(33) 2-[6-oxo-2-phenyl-1,6-dihydro-1-pyrimidinyl]-N-[(1S)-1-[[5-(1-methylcyclopropyl)-1,3,4-oxadiazole-2-yl]carbonyl]-2-methylpropyl]acetamide,
(34) 2-[5-amino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(35) 2-[6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(36) 2-[5-amino-6-oxo-2-(pyridine-3-yl)-1,6-dihydro-1-pyrimidinyl]-N-[(1S)-1-[[5-(t-butyl)-1,3,4-oxadiazole-2-yl]carbonyl]-2-methylpropyl]acetamide,
(37) 2-[5-amino-6-oxo-2-(pyridine-3-yl)-1,6-dihydro-1-pyrimidinyl]-N-[(1S)-1-[[5-(α,α-dimethylbenzyl)-1,3,4-oxadiazole-2-yl]carbonyl]-2-methylpropyl]acetamide,
(38) 2-[5-amino-6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(39) 2-[5-amino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(40) 2-[6-oxo-2-(4-fluorophenyl)-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide,
(41) 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide,
(42) 2-[6-oxo-2-(4-fluorophenyl)-1,6-dihydro-1-pyrimidinyl]-N-[1-[[5-(α,α-dimethylbenzyl)-1,3,4-oxadiazole-2-yl]carbonyl]-2-methylpropyl]acetamide,
(43) 2-[6-oxo-2-phenyl-1,6-dihydro-1-pyrimidinyl]-N-[1-[[5-(α,α-dimethylbenzyl)-1,3,4-oxadiazole-2-yl]carbonyl]-2-methylpropyl]acetamide,
(44) Methoxycarbonyl-L-valyl-N-[(1S)-1-[[5-(t-butyl)-1,3,4-oxadiazole-2-yl]carbonyl]-2-methylpropyl]-L-prolinamide,
(45) Methoxycarbonyl-L-valyl-N-[(1S)-1-[[5-(α,α-dimethylbenzyl)-1,3,4-oxadiazole-2-yl]carbonyl]-2-methylpropyl]-L-prolinamide,
(46) Methoxycarbonyl-L-valyl-N-[(1S)-1-[[5-(α,α-dimethyl-3,4-methylenedioxybenzyl)-1,3,4-oxadiazole-2-yl]carbonyl]-2-methylpropyl]-L-prolinamide,
(47) 2-[5-methoxycarbonylamino-6-oxo-2-phenyl-1,6-dihydro-1-pyrimidinyl]-N-[1-[[5-(t-butyl)-1,3,4-oxadiazole-2-yl]carbonyl]-2-methylpropyl]acetamide,
(48) 1-[(3S)-(2-(t-butoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(49) 1-[(3S)-(2-(1-methylethoxycarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(50) 1-[(3S)-(2-(imidazole-5-ylcarbonyl)-1,2,3,4-tetrahydroisoquinoline-3-yl]-N-[(1S)-1-[[5-(t-butyl)-1,3,4-oxadiazole-2-yl]carbonyl]-2-methylpropyl]carboxamide,
(51) 1-[(3S)-(2-[(1S)-1-(methoxycarbonylamino)-2-methylpropylcarbonyl]-1,2,3,4-tetrahydroisoquinoline-3-yl]-N-[(1S)-1-[[5-(t-butyl)-1,3,4-oxadiazole-2-yl]carbonyl]-2-methylpropyl]carboxamide (CAS Registry No. 251540-46-2),
(52) 1-[(3S)-(2-[(1S)-1-(pyridine-3-ylcarbonylamino)-2-methylpropylcarbonyl]-1,2,3,4-tetrahydroisoquinoline-3-yl]-N-[(1 S)-1-[[5-(t-butyl)-1,3,4-oxadiazole-2-yl]carbonyl]-2-methylpropyl]carboxamide,
(53) 1-[(2S)-1-(t-butoxycarbonyl)-2,3-dihydroindole-2-yl]-N-[(1S)-1-[[5-(t-butyl)-1,3,4-oxadiazole-2-yl]carbonyl]-2-methylpropyl]carboxamide,
(54) 1-[(2S)-1-(2-methylethoxycarbonyl)-2,3-dihydroindole-2-yl]-N-[(1S)-1-[[5-(t-butyl)-1,3,4-oxadiazole-2-yl]carbonyl]-2-methylpropyl]carboxamide,
(55) 1-[(2S)-1-(imidazole-5-ylcarbonyl)-2,3-dihydroindole-2-yl]-N-[(1S)-1-[[5-(t-butyl)-1,3,4-oxadiazole-2-yl]carbonyl]-2-methylpropyl]carboxamide,
(56) 1-[(2S)-1-[(1S)-1-(methoxycarbonylamino)-2-methylpropylcarbonyl]-2,3-dihyd roindole-2-yl]-N-[( 1S)-1-[[5-(t-butyl)-1,3,4-oxadiazole-2-yl]carbonyl]-2-methylpropyl]carboxamide,
(57) 1-[(2S)-1-[(1S)-1-(pyridine-3-ylcarbonylamino)-2-methylpropylcarbonyl]-2,3-dihydroindole-2-yl]-N-[(1S)-1-[[5-(t-butyl)-1,3,4-oxadiazole-2-yl]carbonyl]-2-methylpropyl]carboxamide,
(58) 2-[5-methoxycarbonylamino-6-oxo-2-phenyl-1,6-dihydro-1-pyrimidinyl]-N-[1-[[5-(α,α-dimethyl-3,4-methylenedioxybenzyl)-1,3,4-oxadiazole-2-yl]carbonyl]-2-methylpropyl]acetamide.

8. Prophylactic and/or therapeutic drugs described in claim 1, wherein ulcerative colitis are ulcerative colitis, Crohn's disease, the ischemic colitis or the intestinal Behcet's syndrome.
